# EUROPÄISCHE PATENTSCHRIFT

(11) **EP 2 886 102 B1**
(45) Veröffentlichungstag und Bekanntmachung des Hinweises auf die Patenterteilung: **17.04.2019**
(21) Anmeldenummer: 14195102.0
(22) Anmeldetag: 27.11.2014
(51) Int. Cl.: A61K 8/895, A61K 8/06, C08J 3/03, C08L 83/06

(54) **SILICON(METH-)ACRYLAT-PARTIKEL, VERFAHREN ZU DEREN HERSTELLUNG SOWIE DEREN VERWENDUNG**
SILICON (METH-)ACRYLATE PARTICLE, METHOD FOR THEIR MANUFACTURE AND ITS APPLICATION
PARTICULES DE SILICONE (MÉTH-)ACRYLATE, SON PROCÉDÉ DE FABRICATION ET D'UTILISATION

(30) Priorität: 19.12.2013 DE 102013226568
(43) Veröffentlichungstag der Anmeldung: 24.06.2015
(73) Patentinhaber: Evonik Degussa GmbH, 45128 Essen (DE)
(72) Erfinder: Klostermann, Michael, 45239 Essen (DE); Wiechers, Susann, 45239 Essen (DE); Naumann, Matthias, 22159 Hamburg (DE); Venzmer, Joachim, 45239 Essen (DE)
(74) Vertreter: Evonik Patent Association

(56) Entgegenhaltungen:
- WO-A1-00/31203
- DE-A1-102007 058 713
- BERNARD P BINKS: "Particles as Surfactants --similarities and differences", CURRENT OPINION IN COLLOID AND INTERFACE SCIENCE, LONDON, GB, Bd. 7, Nr. 1-2, 1. März 2002 (2002-03-01), Seiten 21-41, XP002296320, ISSN: 1359-0294, DOI: 10.1016/S1359-0294(02)00008-0

## Beschreibung

Die vorliegende Erfindung betrifft Partikel, erhältlich durch Polymerisation eines Siloxans, das mindestens eine (Meth-)Acrylat-Gruppe der Formel (I)

-O-C(O)-CR=CH₂ (I)

mit R = -H oder -CH₃, aufweist, welche dadurch gekennzeichnet sind, dass das Siloxan ein mittleres molares Verhältnis von Gruppen der Formel (I) zu Si Atomen von kleiner 0,1 aufweist, ein Verfahren umfassend die Schritte: a) Erzeugen einer Emulsion aus einer wässrigen Phase und einer organischen Phase, wobei die organische Phase mindestens ein Siloxan aufweist, das mindestens eine (Meth-)Acrylat-Gruppe der Formel (I) aufweist, unter Zugabe zumindest eines Emulgators, vorzugsweise eines Festkörperemulgators, und Mischen der beiden Phasen, wobei die organische Phase die innere Phase der Emulsion bildet, und b) Auspolymerisieren der inneren Phase in Gegenwart eines Radikalstarters, der in der organischen Phase besser löslich ist als in der wässrigen Phase, welches dadurch gekennzeichnet ist, dass das eingesetzte Siloxan ein mittleres molares Verhältnis von Gruppen der Formel (I) zu Si Atomen von kleiner 0,1 aufweist, entsprechend hergestellte Partikel sowie die Verwendung der Partikel, vorzugsweise in kosmetischen Formulierungen

Es ist bekannt hydrophobe bzw. hydrophobierte Partikel oder Siliconharz-Partikel in kosmetischen Formulierungen zu verwenden, z. B. als Mattierungsmittel, als Absorber für Hautfette oder zur Erzeugung eines seidigen Hautgefühls. Zudem werden entsprechende Partikel oft in Make-up Formulierungen verwendet, um die Haltbarkeit des Make-ups auf der Haut zu verbessern. Häufig werden hierzu Silicon-Gummi-Pulver oder Pulver von Silicon-Elastomeren eingesetzt.

Zur Herstellung entsprechender Partikel sind mehrere Methoden bekannt. Prinzipiell können irregulär geformte Siliconelastomerpartikel durch Vermahlungsprozesse der jeweiligen Bulk-Elastomere gewonnen werden, jedoch bieten sphäroide oder kugelförmige Partikel in der Regel anwendungstechnische Vorteile, vor allem, wenn eine anprechende Haptik der partikeladditivierten Materialien und Formulierungen gewünscht wird. Üblicherweise werden solche Partikel durch Vernetzungsreaktionen innerhalb von Edukt-Tröpfchen oder Aufwachsen/Aufbringung eines Polymers auf einen Partikelkern hergestellt. Vernetzungsreaktionen können dabei z. B. Hydrosilylierungsreaktionen, Kondensationsreaktionen dehydrogenative Kopplungsreaktionen oder radikalische Polymerisation sein.

Siliconpartikel aus Hydrosilylierungen werden beispielsweise in US 4,761,454, JP 2003301047 und EP 1 074 575 beschrieben, Hydrolyse- und Kondensationsreaktionen zur Herstellung von Siliconpartikeln finden sich in EP 1 130 046, WO 2006/016968, JP 2003002973, US 6,753,399, EP 0 765 896 sowie EP 0 744 432, während US 2004/0156808 eine dehydrogenative Kopplungsreaktion zu diesem Zweck beschreibt. Schließlich werden in DE 10 2004 053 314 über radikalische Polymerisationen erhältliche Copolymere aus mindestens zwei verschiedenen Makromonomeren beschrieben.

Über die Herstellung von radikalisch vernetzten Silikonacrylat-Partikeln wurde erstmals 2005 in "Polydimethyl siloxane latexes and copolymers by polymerization and polyaddition in miniemulsion" (Katharina Landfester, Ute Pawelzik, Markus Antonietti, Polymer, 46 (2005), 9892-9898) berichtet. Im Detail wird hier die Miniemulsionspolymerisation von Siliconacrylaten zu nanoskaligen Partikeln unter Verwendung eines herkömmlichen Emulgators und eines gängigen molekularen Radikalstarters, wie beispielsweise AIBN, beschrieben. Jedoch liefert der beschriebene Prozess keine mikroskaligen Partikel, welche die gewünschten anwendungstechnischen Eigenschaften besitzen, z. B. ist mit solchen Partikeln nicht das für Personal Care-Anwendungen gewünschte gute Hautgefühl zu erzielen.

Festkörperstabilisierte, wässrige Emulsionen wurden 1907 von S. U. Pickering beschrieben ("Emulsions", Spencer Umfreville Pickering, Journal of the Chemical Society, Transactions (1907), 91, 2001-2021) und gelten als besonders stabil gegenüber Koaleszenz. So beschreibt DE 10 2004 014 704 beispielsweise die Herstellung von Emulsionen, die mit pyrogen erzeugten Partikeln stabilisiert sind. Eine gute Übersicht über die Eigenschaften solcher stabilisierender Feststoffpartikel findet sich in "Particles as surfactants - similarities and differences" von Bernhard P. Binks (Current opinion in colloid & interface science, 7 (2002), 21-41). Zum Stand der Technik gehören auch sogenannte "Janus-Partikel", amphiphile Partikel mit hemisphärisch modifizierter Oberfläche, wie z. B. in FR 2 808 704 beschrieben. Besonders gut geeignet zur Emulsionsstabilisierung sind nanoskalige, vorwiegend anorganische Partikel, z. B. Silica-Partikel, welche als "LUDOX®" in Form wässriger Sole bzw. Dispersionen von der Fa. Grace Davison im Handel erhältlich sind. In US 3,615,972 (1967) wird erstmals die Verwendung von LUDOX®-Partikeln zur Emulsionsstabilisierung von Methylmethacrylat mit nachfolgender Polymerisation beschrieben. Als Mechanismus der stabilisierenden Wirkung wird in der Literatur die Agglomeration der Partikel und die Anreicherung der Agglomerate an der Wasser/Öl-Grenzfläche diskutiert ("The mechanism of emulsion stabilization by small silica (LUDOX®) particles", Helen Hassander, Beatrice Johansson, Bertil Törnell, Colloids and Surfaces, 40, (1989), 93-105).

Die Suspensionspolymerisation von Pickering-Emulsionen schlecht oder nicht wasserlöslicher Edukte wird üblicher Weise mittels eines in der Ölphase gelösten Radikalstarters gestartet werden, der Einsatz wasserlöslicher Radikalstarter führt, beispielsweise mit Styrol als einzigem Monomer, zu unvollständiger Umsetzung und Koagulation ("Pickering stabilized miniemulsion polymerization: Preparation of clay armored latexes", Severine Cauvin, Patrick J. Colver, and Stefan A. F. Bon, Macromolecules 2005, 38, 7887-7889).

DE 102007058713 beschreibt ein Verfahren zur Herstellung von mikroskaligen Silicon(meth)acrylat-Partikeln durch Suspensionspolymerisation unter der Verwendung eines Festkörperemulguators sowie eines wasserlöslichen Redox-Radikalstarters.

JP 2006-070378 beschreibt einen Prozess zur Herstellung von Dispersionen enthaltend Silicon-(Meth)acrylat-Partikel. Die Herstellung erfolgt unter Verwendung eines Emulgators und eines wasser-löslichen Initiators. JP 2000-063462 beschreibt die Herstellung von hydrophilen Siloxan-Latex-Emulsionen.

Ein Nachteil dieser Verfahren ist, dass hohe Konzentrationen eines wasserlöslichen Radikalstarters notwendig sind, um eine ausreichende Vernetzung der Partikel zu gewährleisten. Darüber hinaus erfordern diese hohen Radikalstarterkonzentrationen den Einsatz großer Mengen eines Kaliumphosphat-Puffers, um den pH-Wert des Systems während der Polymerisation konstant zu halten. Hierdurch kommt es bei diesem Verfahren zu einer ökologisch unerwünschten hohen Salzbelastung des prozessbedingten Abwassers. Darüber hinaus sind aufwendige, kostenintensive Waschzyklen erforderlich, um nach der Polymerisation Radikalstarter- und Pufferreste aus dem Produkt zu entfernen.

Ein weiterer Nachteil der so hergestellten Silicon(meth)acrylat-Partikel ist, dass die polymerisierten Partikel einen relativ hohe Gehalte an nicht abreagierten, aber kovalent an das Polymerisat gebundene, (Meth)acrylatgruppen aufweisen. Diese führen häufig dazu, dass bei Anwesenheit esterspaltender oder umesternder Substanzen, wie beispielsweise Wasser oder Alkoholen, oft ein unangenehmer Geruch wahrnehmbar ist, der die Partikel für verschiedene Anwendungen, insbesondere konsumentennahe, unbrauchbar macht.

Darüber hinaus haben die so hergestellten Partikel den Nachtteil, das sie durch die Einwirkung des Redox-Radikalstarters meist eine gelblich-bräunliche Verfärbung aufweisen, was vor allem für Anwendungen im kosmetischen Bereich unerwünscht ist. Dies hat zur Folge, dass die Herstellung der Partikel oftmals einen zusätzlichen Bleichschritt, beispielsweise mit H₂O₂, erforderlich macht, was sowohl aus ökologischer als auch ökonomischer Sicht ungünstig ist.

Ein weiterer Nachteil der nach DE 102007058713 hergestellten Partikel ist, dass sie irreguläre, nicht-sphärische Strukturen aufweisen, was sich negativ auf das Hautgefühl der Partikel auswirkt.

Neben der Herstellung ist auch die Verwendung verschiedenster silikonbasierter Partikel in kosmetischen Zusammensetzungen in einer Reihe von Schriften beschrieben.

So werden beispielsweise in EP 0834305 gelartige kosmetische Hautbehandlungszusammensetzungen beschrieben, die kugelförmige Pulver von Oganopolysiloxanen mit einer mittleren Partikelgröße von 1 bis 15 µm enthalten. Als weitere Partikel werden insbesondere hydrophobierte bzw. mit Silicon behandelte anorganische Pulver zugesetzt.

In EP 0765656 werden kosmetische Wasser-in-ÖI Emulsionen als kosmetische Zusammensetzungen beschrieben, die Pulver von sphärischen, elastomeren Organopolysiloxan-Partikeln enthalten. Neben den elastomeren (verformbaren) Partikeln weisen diese Zusammensetzungen hydrophobierte Kieselsäure Partikel auf.

In FR 2682534 werden Hautkosmetika beschrieben, die zwei verschiedene Partikelfraktionen aufweisen, wobei die eine Fraktion aus nicht verformbaren Partikeln, vorzugsweise Glasperlen und die andere aus verformbaren, also elastischen Partikeln besteht.

Den genannten Dokumenten ist gemein, dass durch die Verwendung der Elastomerpartikel ein angenehmes (weiches), pudriges Hautgefühl erreicht werden soll.

Über die Verwendung von nicht-elastomeren Silikonacrylat- und Methacrylatpartikeln in kosmetischen Anwendungen wurde erstmals in DE 102007058713 sowie in WO 2012/100884 A1 berichtet. Die in WO 2012/100884 A1 beschriebenen Partikel weisen ein nicht optimales Hautgefühl auf. Darüber hinaus weisen diese Silikon(meth)acrylat-Partikel in den kosmetischen Zusammensetzungen die oben beschriebenen, auf ihr auf einem wasserlöslichen Radikalstarter basierendem Herstellverfahren zurückzuführenden Nachteile auf, insbesondere weisen die Partikel Dimpel (Einfallstellen) auf, was sich nachteilig auf ihr Hautgefühl auswirkt.

Aufgabe der vorliegenden Erfindung war die Bereitstellung von Silicon(meth-)acrylat-Partikeln, die einen oder mehrere Nachteile der Partikel des Standes der Technik nicht aufweisen und sich durch ein besonders vorteilhaftes Hautgefühl auszeichnen.

Überraschenderweise wurde gefunden, dass die Aufgabe gelöst wird durch Partikel, erhältlich durch Polymerisation eines Siloxans, das mindestens eine (Meth-)Acrylat-Gruppe aufweist, wobei das Siloxan ein mittleres molares Verhältnis von (Meth-)Acrylat-Gruppen zu Si Atomen von kleiner 0,1 aufweist. Zudem wurde gefunden, dass Partikel mit einem besonders vorteilhaften Hautgefühl bereitgestellt werden können, wenn zur Herstellung der Partikel ein öllöslicher Radikalstarter verwendet wird, da so eine gleichmäßigere Form der Partikel erzeugt wird.

Gegenstand der vorliegenden Erfindung ist daher ein Verfahren umfassend die Schritte: a) Erzeugen einer Emulsion aus einer wässrigen Phase und einer organischen Phase, wobei die organische Phase mindestens einen Radikalstarter und mindestens ein Siloxan aufweist, das mindestens eine (Meth-)Acrylat-Gruppe der Formel (I)

-O-C(O)-CR=CH₂ (I)

mit R = -H oder -CH₃, aufweist,
unter Zugabe zumindest eines Emulgators, bevorzugt eines Festkörperemulgators und Mischen der beiden Phasen, wobei die organische Phase die innere Phase der Emulsion bildet, und b) Auspolymerisieren der inneren Phase in Gegenwart eines Radikalstarters, der in der organischen Phase besser löslich ist als in der wässrigen Phase, welches dadurch gekennzeichnet ist, dass das eingesetzte Siloxan ein mittleres molares Verhältnis von Gruppen der Formel (I) zu Si Atomen von kleiner 0,1 aufweist.

Ein weiterer Gegenstand der vorliegenden Erfindung sind Partikel, die durch ein solches Verfahren erhältlich sind.

Ebenfalls Gegenstand der vorliegenden Erfindung sind Partikel, erhältlich durch Polymerisation eines Siloxans, das mindestens eine (Meth-)Acrylat-Gruppe der Formel (I)

-O-C(O)-CR=CH₂ (I)

mit R = -H oder -CH₃, aufweist, welche dadurch gekennzeichnet sind, dass das Siloxan ein mittleres molares Verhältnis von Gruppen der Formel (I) zu Si Atomen von kleiner 0,1 aufweist.

Ebenfalls Gegenstand der vorliegenden Erfindung ist die Verwendung der erfindungsgemäßen Partikel allein oder in Mischung mit weiteren Partikeln, Pigmenten und/oder weiteren üblichen Zusatzstoffen in Form von Pulvern oder Dispersionen in kosmetischen oder pharmazeutischen Zubereitungen oder in Pflegemitteln.

Außerdem sind Gegenstand der vorliegenden Erfindung Zusammensetzungen enthaltend die erfindungsgemäßen Partikel oder die erfindungsgemäß hergestellten Partikel.

Im Vergleich mit den in WO 2012/100884 A1 beschriebenen Silicon(meth)acrylat-Partikeln weisen die erfindungsgemäßen Silikon(meth)acrylat-Partikeln den Vorteil auf, dass sie sich durch ein besonders seidig - samtiges Hautgefühl auszeichnen. Dies kann evtl. darauf zurückzuführen sein, dass die Partikel durch die Verwendung eines öllöslichen Radikalstarters eine gleichmäßigere, kugelige Morphologie aufweisen. wodurch sie sich durch verbesserte sensorische Eigenschaften auszeichnen.

Ein weiterer Vorteil der erfindungsgemäßen Silikon(meth)acrylat-Partikel ist, dass sie bedingt durch ihr auf einem öllöslichen Radikalstarter basierendes Herstellverfahren einen geringeren Gehalt an nicht polymerisierten, aber kovalent an das Polymerisat angebundenen (Meth)acrylat-Gruppen enthalten, wodurch die Hydrolysestabilität der Partikel, vor allem in Gegenwart von esterspaltenden oder umesternden Substanzen, wie beispielsweise Wasser oder Alkoholen, deutlich erhöht wird. Dies ist vorteilhaft für Anwendungen in den Bereichen Kosmetik, Lebensmittelverpackung, Medizinprodukte etc. bei denen ein in Kontakt kommen der Partikel mit Wasser und Alkoholen unter Hydrolysebedingungen nicht ausgeschlossen werden kann. So wird hierdurch sowohl die Qualität als auch die Sicherheit entsprechende Produkte gesteigert.

Des Weiteren haben die beschriebenen Silikon(meth)acrylat-Partikel den Vorteil, dass sie bedingt durch ihr Herstellverfahren keine Verfärbungen aufweisen, wodurch nachträglich aufwendige Bleichschritte entfallen. Zudem fallen durch die Verwendung eines öllöslichen Radikalstarters bei der Herstellung der Silikon(meth)acrylat-Partikel weniger wässrige Abfallprodukte an. So ist zum einen die in den wässrigen Abwässern enthaltene Salzfracht geringer als es bei der Verwendung von wasserlöslichen Startern der Fall wäre. Dies hat zudem zur Folge, dass bei der Herstellung weniger Waschzyklen zur Aufreinigung der Partikel von Nöten sind. Alle diese Punkte liefern sowohl verfahrensökologische als auch verfahrensökonomische Vorteile.

Die erfindungsgemäßen Partikel sowie deren Herstellung und Verwendung werden nachfolgend beispielhaft beschrieben, ohne dass die Erfindung auf diese beispielhaften Ausführungsformen beschränkt sein soll.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer oder Polymer) oder geordnet (Blockoligomer oder Blockpolymer) in diesen Verbindungen vorkommen. Werden nachfolgend Angaben in Prozent gemacht, so handelt es sich, wenn nicht anders angegeben um Angaben in Gewichts-%. Werden nachfolgend Mittelwerte angegeben, so handelt es sich, wenn nicht anders angegeben um das Zahlenmittel. Werden nachfolgend Stoffeigenschaften, wie z. B. Viskositäten oder ähnliches angegeben, so handelt es sich, wenn nicht anders angegeben, um die Stoffeigenschaften bei 25 °C. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt. Werden in der vorliegenden Erfindung chemische (Summen-)Formeln verwendet, so können die angegebenen Indizes sowohl absolute Zahlen als auch Mittelwerte darstellen. Bei polymeren Verbindungen stellen die Indizes vorzugsweise Mittelwerte dar.

Das erfindungsgemäße Verfahren umfassend die Schritte:
a) Erzeugen einer Emulsion aus einer wässrigen Phase und einer organischen Phase, wobei die organische Phase mindestens einen Radikalstarter und ein Siloxan aufweist, das mindestens eine (Meth-)Acrylat-Gruppe der Formel (I)

   -O-C(O)-CR=CH₂ (I)

   mit R = -H oder -CH₃, vorzugsweise -CH₃, aufweist,
   unter Zugabe zumindest eines Festkörperemulgators und Mischen der beiden Phasen, wobei die organische Phase die innere Phase der Emulsion bildet, und
b) Auspolymerisieren der inneren Phase in Gegenwart des Radikalstarters, der in der organischen Phase besser löslich ist als in der wässrigen Phase, zeichnet sich dadurch aus, dass das Siloxan ein mittleres molares Verhältnis von Gruppen der Formel (I) zu Si Atomen von kleiner 0,1 ist, vorzugsweise von 0,02 bis 0,08 aufweist.

Vorzugsweise wird ein Siloxan eingesetzt, dass der Formel (II) genügt 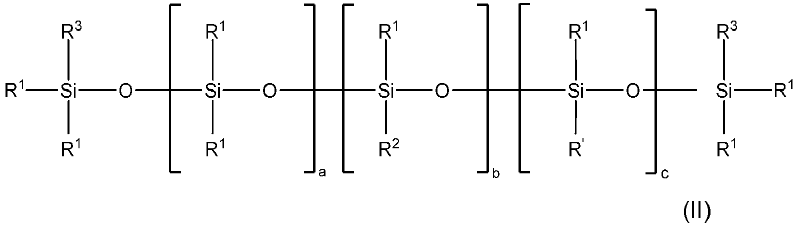 mit
R¹ = gleiche oder verschiedene Alkylreste, vorzugsweise Methylreste,
R² = gleiche oder verschiedene Reste ungleich R', R¹ und R³, die Kohlenstoffatome aufweisen,
R³ = gleiche oder verschiedene Reste R¹ oder Reste, die mindestens eine Gruppe gemäß Formel (I) aufweisen,
R'= gleiche oder verschiedene Reste, die mindestens eine Gruppe gemäß Formel (I) aufweisen,
a = 9 bis 250,
b = 0 bis 20, vorzugsweise 0 oder 1, bevorzugt 0,
c = 0 bis 20,
und der Maßgabe, dass pro Siloxan der Formel (II) wenn c = 0 mindestens ein Rest R³ vorhanden ist, der mindestens eine Gruppe gemäß Formel (I) aufweist. Die Zahlenwerte für a, b und c stellen vorzugsweise statistische Mittelwerte dar.

Die Reste R² können neben Kohlenstoffatomen noch ein oder mehrere Wasserstoff-Sauerstoff- und Siliziumatome aufweisen. Vorzugsweise weisen die Reste R² keine Siliziumatome auf. Bevorzugte Reste R² sind solche die eine Acetatgruppe aufweisen. Besonders bevorzugte Reste R² sind solche, die der Formel -(CH₂)₃-O-CH₂-CH(OH)-CH₂-O-C(O)-CH₃ genügen.

Vorzugsweise wird in dem erfindungsgemäßen Verfahren mindestens ein Siloxan der Formel (II) eingesetzt wird, bei dem a einen Wert von 50 bis 220, bevorzugt von 75 bis 180 einnimmt.

Das in dem erfindungsgemäßen Verfahren eingesetzte Siloxan ist vorzugsweise ein Siloxan der Formel (II), bei dem c einen Wert von 0 oder 4 bis 12 einnimmt, und alle Reste R³ solche Reste sind, die eine Gruppe gemäß Formel (I) aufweisen.

Bevorzugt in dem erfindungsgemäßen Verfahren eingesetzte Siloxane sind solche der Formel (II), bei denen a einen Wert von 75 bis 180 und c einen Wert von 4 bis 12 einnimmt oder a einen Wert von 50 bis 130 und c = 0 und dabei alle Reste R³ solche Reste sind, die eine Gruppe gemäß Formel (I) aufweisen. Der Wert für b ist bei diesen bevorzugten Ausführungsformen vorzugsweise 0.

Als Silicon(meth-)acrylate können beispielsweise die bei der Evonik Industries AG erhältlichen Produkte TEGO® RC 711,726, 902 bzw. deren Methacrylat-Varianten eingesetzt werden.

Besonders bevorzugt in dem erfindungsgemäßen Verfahren eingesetzte Siloxane, die mindestens eine (Meth-)Acrylat-Gruppe der Formel (I) aufweisen, sind solche, die diese in Form eines Restes aus der Gruppe der Reste 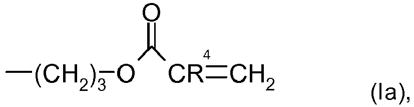 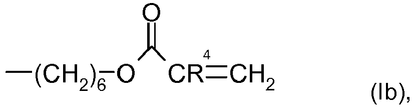 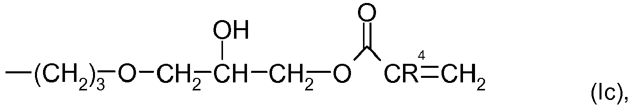 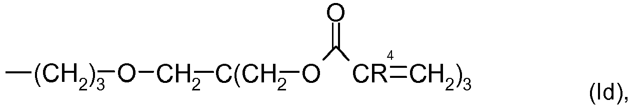 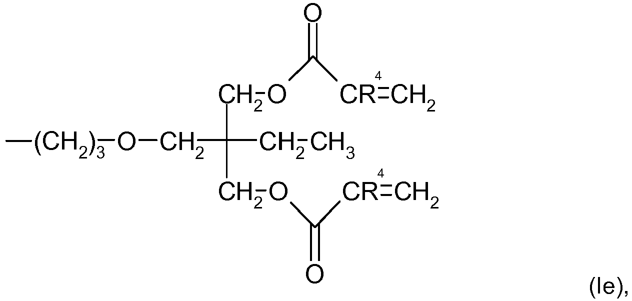 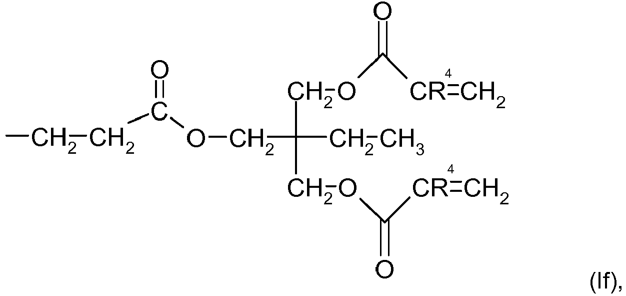 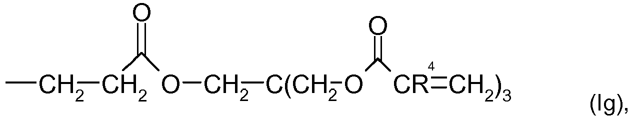 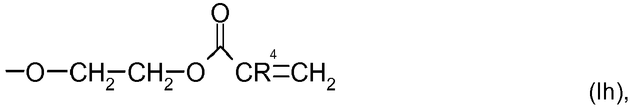 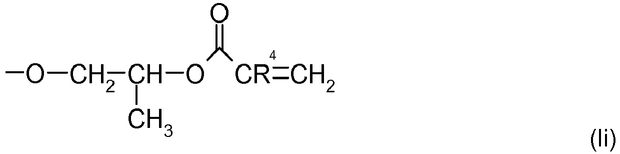 und/oder 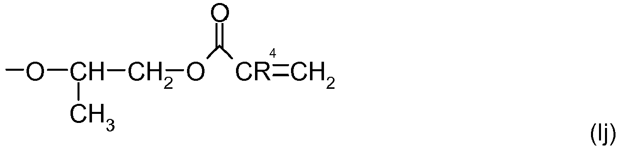 aufweisen, wobei R⁴ Wasserstoff oder eine Methylgruppe ist, vorzugsweise eine Methylgruppe ist. Ganz besonders bevorzugte Siloxane, die mindestens eine (Meth-)Acrylat-Gruppe der Formel (I) aufweisen, sind solche, die diese in Form eines Restes ausgewählt aus der Gruppe umfassend die Reste mit den Formeln (Ib) und (Ie) aufweisen, wobei R⁴ eine Methylgruppe ist.

Im erfindungsgemäßen Verfahren können die Siloxane enthaltend mindestens eine (Meth-)Acrylat-Gruppe der Formel (I), insbesondere die der Formel (II) einzeln oder als Mischungen, insbesondere als statistische Mischungen eingesetzt werden. Vorzugsweise werden in dem erfindungsgemäßen Verfahren Mischungen von Siloxane, insbesondere solche der Formel (II) eingesetzt, in denen die Siloxane sich bezüglich ihrer Struktur und/oder ihrem Molekulargewicht unterscheiden.

Durch den Einsatz von (partikulären) Festkörperemulgatoren können mit dem erfindungsgemäßen Verfahren Partikel, insbesondere solche des Kern-Schale-Typs, hergestellt werden. Diese weisen im Kern das polymerisierte Siloxan und als Schale die partikulären Emulgatoren auf.

Es kann vorteilhaft sein, wenn der organischen Phase vor Schritt b) Comonomere, insbesondere ethylenisch oder vinylisch ungesättigte Gruppen aufweisende Comonomere zugefügt werden. Solche Comonomere können z. B. ein- oder mehrfach (meth-)acrylierte, organische Mono- oder Oligomere, wie sie beispielsweise auch unter den Gruppennamen LAROMER® (BASF AG), EBECRYL® (Cytec Surface Specialties)oder DESMOLUX® (Bayer Material Science) im Handel sind, sein. Bevorzugt werden hierbei (Meth)acrylate von langkettigen Fettalkohlen, wie z.B. Laurylmethacrylat, Myristylmethacrylat, Cetymethyacrylat oder Stearylmetharclat verwendet. Unter ethylenisch ein- oder mehrfach ungesättigten organischen Mono- oder Oligomeren sollen auch ethylenisch ungesättigte, bevorzugt vinylische Gruppen tragende Organopolysiloxane verstanden werden. Vorzugsweise erfolgt die Zugabe der weiteren Comonomere vor Schritt a).

Die organischen Comonomere können nach abgeschlossener Polymerisation in Schritt b) sowohl in das Polysiloxan-(meth)acrylat-Netzwerk kovalent einreagiert sein als auch als eigenständiges Netzwerk vorliegen. Ebenso sind Mischformen dieser beschriebenen Grenzfälle möglich und Teil dieser Erfindung.

Als Festkörperemulgatoren werden in dem erfindungsgemäßen Verfahren vorzugsweise solche eingesetzt, die ausgewählt sind aus der Gruppe der (Halb-)Metalloxide, Mischoxide, Nitride, Hydroxide, Carbonate oder Silikate. Bevorzugte Festkörperemulgatoren weisen Metalloxid und/oder ein Halbmetalloxid auf. Besonders bevorzugte Festkörperemulgatoren basieren auf Siliziumdioxid.

Um die Ausbildung einer Emulsion zu unterstützten, bei der die innere Phase die organische Phase ist, kann es vorteilhaft sein, in dem erfindungsgemäßen Verfahren solche Festkörperemulgatoren einzusetzen, die an der Phasengrenze Wasser/organische Phase einen Kontaktwinkel von kleiner 90° aufweisen. Der Kontaktwinkel kann, wie von Bernard Paul Binks, Lucio Isa und Andrew Terhemen Tyowua in "Direct Measurement of Contact Angles of Silica Particles in Relation to Double Inversion of Pickering Emulsions" in Dokument dx.doi.org/10.1021/Ia4006899 Langmuir 2013, 29, 4923-4927 beschrieben, bestimmt werden.

Es kann vorteilhaft sein, wenn Festkörperemulgatoren eingesetzt werden, die hydrophobiert oder teilhydrophobiert sind. Als Hydrophobierungsmittel eigenen sich z. B. Verbindungen aus der Gruppe der Silane, Siloxane, quaternären Ammoniumverbindungen, kationischen Polymere und Fettsäuren oder deren Anionen. Werden teilhydrophobierte Festkörperemulgatoren eingesetzte werden vorzugsweise solche verwendet, die keine Janus-Partikel sind (sondern eine gleichmäßige Verteilung der hydrophobierten und nicht-hydrophobierten Bereiche auf der Oberfläche des Festkörperemulgators aufweisen). Besonders bevorzugte Festkörperemulgatoren weisen Siliziumdioxid auf und sind zumindest teilhydrophobiert, wobei teilhydrophobierte Partikel bevorzugt sind, die keine Janus-Partikel sind.

Die in dem erfindungsgemäßen Verfahren eingesetzten Festkörperemulgatoren weisen vorzugsweise eine mittlere (volumengemittelt) Partikelgröße (Primärpartikelgröße) von > 100 nm bis < 200 nm auf. Die Partikelgröße der Festkörperemulgatoren, kann auf bekannte Weise bestimmt werden. Vorzugsweise wird die mittlere Primärpartikelgröße durch dynamische Lichtstreuung ermittelt. Hierfür kann beispielsweise der ZetaSizer Nano ZSP der Firma Malvern verwendet werden. Alternativ kann die Partikelgröße durch die optische Auswertung einer durch Transmissionselektronenmikroskopie erstellten Aufnahme bestimmt.

Die partikulären Emulgatoren (Festkörperemulgatoren) können in dem erfindungsgemäßen Verfahren als solche oder in Form von Dispersionen oder Solen, insbesondere wässrigen Dispersionen oder Solen eingesetzt werden.

Die Menge der eingesetzten partikulären Emulgatoren beträgt vorzugsweise 2 bis 30 Gew.-%, bevorzugt 5 bis 25 Gew.-%, besonders bevorzugt 7,5 bis 20 Gew.-% bezogen auf die eingesetzten Siloxane, enthaltend mindestens eine (Meth)acrylat-Gruppe der Formel (I).

Es kann vorteilhaft sein, wenn in Schritt a) des erfindungsgemäßen Verfahrens die Herstellung der Emulsion unter Zugabe eines oder mehrerer Coemulgatoren durchgeführt wird. Als Coemulgatoren können in dem erfindungsgemäßen Verfahren insbesondere solche Verbindungen eingesetzt werden, die mit den Festkörperemulgator-Partikeln in Wechselwirkung treten, vorzugsweise solche, die durch elektrostatische Wechselwirkung gleichmäßig auf die Oberfläche der Festkörperemulgator-Partikel aufziehen und so zu einer (teilweisen) Hydrophobierung der Emulgatorpartikel führen. In dem erfindungsgemäßen Verfahren können als Coemulgatoren insbesondere Verbindungen ausgewählt aus der Gruppe der kationischen Tenside eingesetzt werden. Als kationische Coemulgatoren können insbesondere kationische Ammoniumverbindungen eingesetzt werden. Solche Verbindungen sind z. B. unter den Handelsnamen VARISOFT® 470 P, VARISOFT® TC-90, VARISOFT® 110, VARISOFT® TA-100, ADOGEN® 442-100 P, ADOGEN® 432, ADOGEN® 470, ADOGEN® 471, ADOGEN® 464, VARIQUAT® K 300, VARIQUAT® B 343, VARIQUAT® 80 ME, REWOQUAT® 3690, REWOQUAT® WE 15, REWOQUAT® WE18, REWOQUAT® WE 28 oder REWOQUAT® CR 3099 bei der Evonik Industries AG erhältlich. Bevorzugt wird in dem erfindungsgemäßen Verfahren VARISOFT® TA-100 oder VARISOFT® PATC (beide erhältlich bei der Evonik Industries AG), besonders bevorzugt VARISOFT® PATC als kationischer Coemulgator eingesetzt. Ganz besonders bevorzugt werden in dem erfindungsgemäßen Verfahren solche Silicon(meth)acrylat-Partikel hergestellt, bei deren Herstellung kein Cetyltrimethylammoniumbromid oder -chlorid eingesetzt wird/wurde.

Optional können der organischen Phase in Schritt a) weitere Komponenten zugefügt werden. Die weiteren Komponenten können in der organischen Phase oder der Mischung in Schritt a) gelöst oder dispergiert sein. Bezogen auf die organische Phase können die weiteren Komponenten vorzugsweise in einer Konzentration von 0,01 bis 99 Gew.-%, bevorzugt, 0,1 bis 80 Gew.-% und besonders bevorzugt von 1 bis 50 Gew.-% vorliegen. Solche weiteren Komponenten können funktionelle Komponenten oder nichtfunktionelle Komponenten sein. Als weitere Komponenten können insbesondere eindispregierbare Feststoffe, wie z. B. anorganische Partikel und/oder Fasern, wie z. B. solche der Metalloxide, Mischoxide, Nitride, Hydroxide, Carbonate, Silikate, Pigmente, Ruße, Elemente oder Legierungen, und/oder organische Partikel und/oder Fasern, wie z. B. solche aus Siliconharzen, Siliconen oder organische Polymere oder Biopolymeren, eingesetzt werden, vorzugsweise mit der Maßgabe, dass die Füllstoffe von den eingesetzten Emulgatoren verschieden sind. Eindispergierbare Feststoffe können beispielsweise Fällungskieselsäure, Diatomeenerde (Kieselgur), pyrogene Kieselsäure, Quarzmehl, Titandioxid, Zinkoxid, Ceroxid, Eisenoxid, Ruß, Graphit, Kohlenstoff-Nanoröhren oder -fasern, Alumosilikate, Erdalkalicarbonate, Aluminiumtrihydroxid, Magnesiumdihydroxid bzw. andere aus dem Stand der Technik bekannte und übliche Feststoffe sowie jede der genannten Substanzen nach Oberflächenmodifizierung mit Organosiliziumverbindungen wie Trimethylchlorsilan, Hexamethyldisilazan, (Meth-)Acryloxypropyltrialkoxysilanen, Aminopropyltrialkoxysilanen, Polydimethylsiloxanen, Polysiloxanen, die Si-H-Gruppen tragen, oder reinen Carbonsäuren, Chelatbildnern oder Fluoropolymeren, sein. Diese Feststoffe können beispielsweise als Füllstoffe zur Erzielung bestimmter mechanischer Eigenschaften, als UV-Schutzmittel, als Pigmente, als Antistatik-Zusätze oder zur Erzielung ferromagnetischer Eigenschaften dienen.

In dem erfindungsgemäßen Verfahren kann die organische Phase auch Substanzen beinhalten, welche ggf., vorzugsweise über einen längeren Zeitraum hinweg, aus den Partikeln freigesetzt werden können. Solche Substanzen können z. B. kosmetische Öle und Wirkstoffe, Duftstoffe, pharmazeutische Wirkstoffe, kosmetische Aktivstoffe, antimikrobielle Wirkstoffe, auch zum Beispiel Silber und silberhaltige Verbindungen, sowie Farb- und Konservierungsstoffe sein.

Es kann vorteilhaft sein, wenn in Schritt a) des erfindungsgemäßen Verfahrens die in Schritt a) erzeugte Emulsion 10 bis 40 Gew.-% organische Phase, besonders bevorzugt 15 bis 35 Gew.-% bezogen auf die Emulsion enthält.

Es kann vorteilhaft sein, wenn in Schritt a) des erfindungsgemäßen Verfahrens eine Emulsion erzeugt wird, deren mittlere Tröpfchengröße von 0,01 bis 1.000 µm, bevorzugt 0,1 bis 500 µm und besonders bevorzugt von 1 bis 100 µm eingestellt wird.

Besonders vorteilhaft kann es sein, wenn in Schritt a) des erfindungsgemäßen Verfahrens die in Schritt a) erzeugte Emulsion 10 - 40 Gew.-% organische Phase, besonders bevorzugt 15 - 35 Gew.-% bezogen auf die Emulsion enthält, und die Emulsion eine mittlere Tröpfchengröße von 0,01 bis 1.000 µm, bevorzugt 0,1 bis 500 µm und besonders bevorzugt von 1 bis 100 µm aufweist.

Die Tröpfchengröße kann unter Zuhilfenahme der Lichtmikroskopie (bis ca. 1 µm als untere Grenze) durch Ausmessen des jeweils kleinsten und größten Tröpfchendurchmessers im Sichtfeld abgeschätzt werden, es sollten sich dabei mindestens 10 x 10 Tropfen im Sichtfeld befinden. Weiterhin ist es möglich, die Tröpfchengrößenverteilungen durch die dem Fachmann geläufigen Methoden der statischen und der dynamischen Lichtstreuung zu bestimmen. Hierfür kann beispielsweise der MasterSizer 3000 der Firma Malvern verwendet werden. Dies gilt auch für Dispersionen auspolymerisierter Partikel, darüber hinaus ist die Partikelgröße durch elektronenmikroskopische Aufnahmen über REM oder TEM bestimmbar und dem Fachmann geläufig.

Vorzugsweise wird die Emulsion in Schritt a) durch Durchleiten des Gemisches enthaltend organische und wässrige Phase durch und Dispergieren des Gemisches in wenigstens einer Interaktionskammer, vorzugsweise mit einer Kapillardicke (Innendurchmesser) von 50 bis 500 µm, und vorzugsweise bei einem Druck von 50 bis 1.000 bar, bevorzugt 100 bis 800 bar, besonders bevorzugt 200 bis 600 bar und anschließende Entspannung des Gemisches auf Umgebungsdruck, z. B. in ein Auslass-Reservoir, hergestellt. Dabei wird vorzugsweise eine der oben genannten bevorzugten Tröpfchengrößen eingestellt. Es kann vorteilhaft sein, wenn zwei oder mehr in Reihe geschaltete Interaktionskammern eingesetzt werden. Auf diese Weise kann die gewünschte Tröpfchengröße leichter eingestellt werden. Die Herstellung von Emulsionen in Interaktionskammern wird ausführlich in US 2004-0063818 bzw. DE 100 11 564 beschrieben, auf die ausdrücklich verwiesen wird. Ein geeignetes Gerät zur Herstellung der Emulsionen wird beispielsweise unter dem Namen Mikrofluidizer von der Firma Microfluidics angeboten.

Um eine Emulsion mit Tröpfchengrößen im bevorzugten Bereich zu erhalten, deren Tröpfchen vorzugsweise eine kugelige Morphologie aufweisen, kann es bei der Zugabe von Coemulgatoren vorteilhaft sein, den Coemulgator bzw. die Coemulgatoren erst zuzugeben, nachdem in einem Teilschritt a1) eine Voremulsion V1 hergestellt wurde. Diese Voremulsion V1 z. B. kann dadurch erhalten werden, dass eine Mischung aus Siloxanen enthaltend (Meth-)acrylat-Gruppen der Formel (I), Wasser und Emulgator, vorzugsweise partikulärem Emulgator und besonders bevorzugt nanopartikulärem SiO₂ und ganz besonders bevorzugt IDISIL Si5530 der Fa. Evonik Industries AG unter Aufbringung starker Scherkräfte, wie dies z. B. mit einem Rotor-Rotor-System möglich ist, emulgiert wird. Ein geeignetes Rotor-Rotor-System wird z. B. als Co-Twister Homogenizer von der Firma Symex angeboten.

Besonders vorteilhaft kann es sein, wenn vor der Herstellung der Voremulsion V1 in einem Teilschritt a0) der für die spätere Reaktion benötigte Radikalstarter in dem verwendeten Siloxanen enthaltend (Meth-)acrylat-Gruppen der Formel (I) gelöst wird. Hier können gegebenenfalls geeignete Lösevermittler verwendet werden.

Zur Stabilisierung der Voremulsion V1 wird dieser in einem weiteren Teilschritt a2) der Coemulgator zugegeben. Die Coemulgatoren können als Reinstoff oder in Form einer Lösung, z. B. einer wässrigen Lösung zugegeben werden. Durch die Zugabe des Coemulgators zur Voremulsion V1 kann die Tröpfchengröße der in der Voremulsion V1 enthaltenen Tropfen quasi eingefroren werden. Durch den Zeitpunkt der Zugabe des Cotensids kann somit Tröpfchengrößenverteilung eingestellt werden. Unter anderem durch die Zugabe-Menge von Emulgator und Coemulgator kann die Tröpfchengrößenverteilung der Emulsion voreingestellt werden. Vorzugsweise beträgt das Gewichts-Verhältnis von partikulärem Emulgator zu Coemulgatoren von 100 zu 1 bis 1 zu 1, bevorzugt von 50 zu 1 bis 3 zu 1.

Die in Schritt a2) erhaltene stabilisierte Voremulsion V2 wird anschließend in Schritt a3) in einem Homogenisator mit Interaktionskammer dispergiert. Vorzugsweise wird die Emulsion in Schritt a) durch Durchleiten des Gemisches enthaltend organische und wässrige Phase durch und Dispergieren des Gemisches in wenigstens einer Interaktionskammer, vorzugsweise mit einer Kapillardicke (Innendurchmesser) von 50 bis 500 µm, und vorzugsweise bei einem Druck von 50 bis 1.000 bar, bevorzugt 100 bis 800 bar, besonders bevorzugt 200 bis 600 bar und anschließende Entspannung des Gemisches auf Umgebungsdruck, z. B. in ein Auslass-Reservoir, hergestellt. Dabei wird vorzugsweise eine der oben genannten bevorzugten Tröpfchengrößen eingestellt. Es kann vorteilhaft sein, wenn zwei oder mehr in Reihe geschaltete Interaktionskammern eingesetzt werden. Auf diese Weise kann die gewünschte Tröpfchengröße besonders einfach eingestellt werden. Ein geeigneter Homogenisator wird beispielsweise unter dem Namen Mikrofluidizer von der Firma Microfluidics angeboten.

Bevorzugt werden in Schritt a3) des erfindungsgemäßen Verfahrens Interaktionskammern eingesetzt, von denen zumindest eine eine Kapillardicke von 100 bis 300 µm aufweist. Besonders bevorzugt werden in Schritt a) des erfindungsgemäßen Verfahrens Interaktionskammern eingesetzt, von denen mindestens eine, vorzugsweise alle, mindestens einen Umlenkknick aufweisen.

Durch die Durchführung der Teilschritte a1) bis a3) und die Verwendung eines Homogenisators mit Interaktionskammer in Teilschritt a3) lassen sich besonders einfach kugelige Tröpfchen mit einer gewünschten Tröpfchengrößenverteilung herstellen.

Die Polymerisation der in Verfahrensschritt a) hergestellten Emulsion wird in Schritt b) durch einen der Emulsion zugesetzten Radikalstarter initiiert, welcher dadurch gekennzeichnet ist, dass er in der organischen Phase besser löslich ist als in der wässrigen Phase. Der Radikalstarter wird vorzugsweise in einer Konzentration von 0,05 bis 2 Gew.-%, bevorzugt 0,1 bis 1 Gew.-% und besonders bevorzugt >0,15 bis 0,6 Gew.-%, bezogen auf die innere (organische) Phase zugefügt. Wie oben beschrieben kann es vorteilhaft sein, wenn der in Verfahrensschritt b) verwendete Radikalstarter der organischen Phase bereits vor oder während Verfahrensschritt a) zugegeben wird. Das Auspolymerisieren erfolgt vorzugsweise in Form einer Suspensionspolymerisation.

Als Radikalstarter können übliche als Radikalstarter geeignete Verbindungen, vorzugsweise organische Radikalstarter eingesetzt werden. Bevorzugt eingesetzte Radikalstarter sind solche, die durch Zuführung von thermischer Energie Radikale bilden. Besonders bevorzugt eingesetzte Radikalstarter sind solche, die nach dem Radikalstart zu Verbindungen werden, die in kosmetischen Produkten verträglich sind, wie z. B. Kohlenwasserstoffe, Alkohole oder Säuren mit 8 bis 30 Kohlenstoffatomen. Als Radikalstarter können insbesondere Peroxide eingesetzt werden, die sich von organischen Säuren, insbesondere Fettsäuren ableiten. Unter diesen, auch als Diacylperoxide bekannten Verbindungen, sind Lauroylperoxid (Dilauroylperoxid), Decanoylperoxid und Isononanoylperoxid bevorzugt. Besonders bevorzugt wird als Radikalstarter Dilauroylperoxid eingesetzt. Die Initiierung erfolgt vorzugsweise durch Temperaturerhöhung. Als Radikalstarter werden bevorzugt keine Redox-Systeme eingesetzt. Gegebenenfalls kann es vorteilhaft sein, wenn die Radikalstarter in Kombination mit Radikalüberträgern eingesetzt werden. Bevorzugte Radikalüberträger können z. B. Acetylaceton, Aceton oder ähnliches sein. Solche Systeme sind als Radikalstarter gut bekannt und im Bereich der Emulsionspolymerisation Stand der Technik.

Der Verfahrensschritt b) wird vorzugsweise bei erhöhter Temperatur durchgeführt. Vorzugsweise wird der Verfahrensschritt b) unter Rühren und bevorzugt unter Schutzgas/Inertisierung durchgeführt. Ansonsten kann die Polymerisation in Schritt b) wie im Stand der Technik beschrieben auf herkömmliche Weise durchgeführt werden.

Nach der Durchführung des Polymerisationsschrittes b) kann es vorteilhaft sein, die erhaltenen Partikel aus der Suspension abzutrennen. Dazu kann z. B. das Wasser nach üblichen Methoden, beispielsweise durch Filtrieren oder Zentrifugieren, entfernt werden. Um den Trocknungsvorgang zu beschleunigen kann es vorteilhaft sein, die Partikel in einem der Waschzyklen z. B. mit Ethanol zu waschen.

Es kann vorteilhaft sein, wenn die Partikel nach der Synthese oberflächenmodifiziert werden. Die Oberflächenmodifizierung kann nach den üblichen Methoden erfolgen. Die Oberflächenmodifizierung kann mit organische und/oder anorganischen sowie mit geladenen und/oder ungeladenen Stoffen durchgeführt werden.

Besonders vorteilhaft kann es sein, wenn das Modifizierungsmittel mindestens eine funktionelle Gruppe aufweist, die mit der zu modifizierenden Oberfläche eine kovalente, ionische oder koordinative Bindung oder Wasserstoffbrückenbindungen eingehen kann.

Neben der mindestens einen funktionelle Gruppe, die mit der Oberfläche des Kern-Schale-Siliconpartikels eine Bindung eingehen kann, kann das Modifizierungsmittel zusätzlich weitere Reste aufweisen, welche die Eigenschaften des Partikels modifizieren. Solche Reste, oder auch Teile davon, können beispielsweise hydrophob oder hydrophil sein oder eine oder mehrere funktionelle Gruppen tragen, um auf diese Weise die Siliconpartikel kompatibel zum umgebenden Medium zu machen.

Die erfindungsgemäßen Partikel zeichnen sich dadurch aus, dass das (eingesetzte) Siloxan ein mittleres molares Verhältnis von Gruppen der Formel (I) zu Si Atomen von kleiner 0,1 aufweist. Die erfindungsgemäßen Partikel sind erhältlich durch ein Verfahren umfassend die Schritte: Erzeugen einer Emulsion aus einer wässrigen Phase und einer organischen Phase, wobei die organische Phase mindestens ein Siloxan aufweist, das mindestens eine (Meth-)Acrylat-Gruppe der Formel (I)

-O-C(O)-CR=CH₂ (I)

mit R = -H oder -CH₃, aufweist,
unter Zugabe zumindest eines Festkörperemulgators und Mischen der beiden Phasen, wobei die organische Phase die innere Phase der Emulsion bildet, und Auspolymerisieren der inneren Phase in Gegenwart eines Radikalstarters, der in der organischen Phase besser löslich ist als in der wässrigen Phase, welches dadurch gekennzeichnet ist, dass das Siloxan ein mittleres molares Verhältnis von Gruppen der Formel (I) zu Si Atomen von kleiner 0,1 aufweist, erhältlich sind, insbesondere durch das vorstehend beschriebene erfindungsgemäße Verfahren.

Die erfindungsgemäßen Partikel weisen vorzugsweise ein Polymer auf, welches durch Polymerisation von Siloxanen enthaltend mindestens eine (Meth-)acrylat-Gruppe der Formel (I) und ggf. anderen Monomeren ggf. in Gegenwart weiterer Komponenten wie beispielsweise Füllstoffen, Hilfs- oder Aktivstoffen etc. in Gegenwart eines Radikalstarters, insbesondere eines organischen Radikalstarters erhalten wurde.

Die erfindungsgemäßen Partikel weisen vorzugsweise eine mittlere Partikelgröße d₅₀ von 1 bis 40 µm, bevorzugt von 3 bis 20 µm, besonders bevorzugt 5 bis 15 auf. Bevorzugte Partikel weisen eine Partikelgröße d₉₀ von 10 bis 100 µm, bevorzugt von 15 bis 80 µm, besonders bevorzugt 40 bis 70 µm auf. Die Partikelgröße d₁₀ der erfindungsgemäßen Partikel beträgt vorzugsweise von 0,5 bis 15 µm, bevorzugt von 1 bis 10 µm, besonders bevorzugt 2 bis 7,5 µm. Ganz besonders bevorzugte erfindungsgemäße Partikel sind solche, die alle der oben genannten Werte für d₁₀, d₅₀ und d₉₀ erfüllen, insbesondere die als am meisten bevorzugt angegebenen Werte. Die Bestimmung der Werte d₁₀, d₅₀ bzw. d₉₀ erfolgt vorzugsweise durch dem Fachmann geläufige statische Laserbeugungsmethoden. Hierfür kann Beispielsweise der MasterSizer 3000 mit angeschlossener Aero S Trockendispergiereinheit der Firma Malvern verwendet werden.

Die erfindungsgemäßen Partikel können nicht abreagierte aber kovalent an das Polymerisat gebundene (Meth)acrylatverbindungen (Verbindungen die eine freie Gruppe der Formel (I) aufweisen) aufweisen, vorzugsweise in einer Konzentration von weniger als 5000 Massen-ppm (wppm), bevorzugt weniger als 3000 Massen-ppm, besonders bevorzugt weniger als 1500 Massen-ppm bezogen auf den Partikel, auf. Der Anteil an nicht abreagierten (Meth)acrylatverbindungen lässt sich beispielsweise durch Hydrolyse der Partikel in ethanolischer Kalilauge und anschließender Bestimmung der freigesetzten (Meth)Acrylsäure mittels HPLC bestimmen.

Bevorzugte erfindungsgemäße Partikel sind solche, die eine Kern-Schale-Struktur besitzen (so genannte Silicon(meth-)acrylat-Komposit-Partikel). Bei diesen umschließt eine Schale, wobei die Schale vorzugsweise durch partikuläre Emulgatoren gebildet wird, den inneren Kern, der das polymerisierte Silicon(meth-)acrylat aufweist. Besonders bevorzugte Silicon(meth-)acrylat-Partikel sind solche, bei denen die Schale aus den oben genannten anorganischen Partikeln gebildet wird, deren Oberfläche vorzugsweise modifiziert ist.

Bevorzugte Partikel können solche sein, bei denen die Schale modifiziert ist. Eine solche Modifizierung kann z. B. mit kationischen Substanzen, wie organischen Ammonium-Ionen oder kationischen Polymeren, kationischen Siloxanen, organischen Polymeren, wie beispielsweise Polyacrylaten, Carbonsäuren oder Carbonsäureanionen, Chelatbildnern, Diketonen, Siloxanen oder kondensierten Silanen wie oben beschrieben erfolgt sein. Dabei kann die Oberflächenmodifizierung physikalisch oder chemisch an das Polymerpartikel gebunden sein. Weiterhin können die Oberflächenmodifikatoren funktionelle Gruppen tragen, wie beispielsweise bei der Verwendung funktioneller Silane. Die Oberflächenmodifikatoren können aus diskreten Molekülen bestehen, aber auch quervernetzt sein.

Neben dem Silicon(meth-)acrylat können die Partikel ggf. bei der Polymerisation eingesetzte Comonomere aufweisen. Diese Comonomeren können vollständig oder teilweise in das Polysiloxan-(meth)acrylat-Netzwerk einreagiert sein oder auch als eigenständiges Netzwerk vorliegen. Ebenso sind Mischformen dieser beschriebenen Grenzfälle möglich und Teil dieser Erfindung.

Es kann vorteilhaft sein, wenn die Partikel weitere Komponenten aufweisen, die keine Bestandteile sind, die aus den Emulgatoren, Monomeren oder Comonomeren hervorgegangen sind. Bezogen auf die Polysiloxan-(meth)acrylatpolymere weisen die Partikel diese weiteren Komponenten vorzugsweise in einer Konzentration von 0,01 bis 99 Gew.-%, bevorzugt von 0,1 bis 80 Gew.-% und besonders bevorzugt von 1 bis 50 Gew.-% auf. Solche weiteren Komponenten können funktionelle Komponenten oder nichtfunktionelle Komponenten sein. Als weitere Komponenten können insbesondere Feststoffe, wie z. B. anorganische Partikel und/oder Fasern, wie z. B. solche der Metalloxide, Mischoxide, Nitride, Hydroxide, Carbonate, Silikate, Pigmente, Ruße, Elemente oder Legierungen, und/oder organische Partikel und/oder Fasern, wie z. B. solche aus Siliconharzen, Siliconen oder organische Polymere oder Biopolymeren, vorzugsweise mit der Maßgabe, dass die Füllstoffe von den eingesetzten Emulgatoren verschieden sind, in den Partikeln enthalten sein. Weitere Komponenten können insbesondere ausgewählt sein aus Fällungskieselsäure, Diatomeenerde (Kieselgur), pyrogene Kieselsäure, Quarzmehl, Titandioxid, Zinkoxid, Ceroxid, Eisenoxid, Ruß, Graphit, Kohlenstoff-Nanoröhren oder -fasern, Alumosilikate, Erdalkalicarbonate, Aluminiumtrihydroxid, Magnesiumdihydroxid bzw. andere aus dem Stand der Technik bekannte und übliche Feststoffe sowie jede der genannten Substanzen nach Oberflächenmodifizierung mit Organosiliziumverbindungen wie Trimethylchlorsilan, Hexamethyldisilazan, (Meth-)Acryloxypropyltrialkoxysilanen, Aminopropyltrialkoxysilanen, Polydimethylsiloxanen, Polysiloxanen, die Si-H-Gruppen tragen, oder reinen Carbonsäuren, Chelatbildnern oder Fluoropolymeren. Diese Feststoffe können beispielsweise als Füllstoffe zur Erzielung bestimmter mechanischer Eigenschaften, als UV-Schutzmittel, als Pigmente, als Antistatik-Zusätze oder zur Erzielung ferromagnetischer Eigenschaften dienen.

Die weiteren Komponenten können nachträglich den bereits polymerisierten Partikeln durch Quellung und Diffusion zugesetzt werden. Dies kann auch unter Zuhilfenahme eines Lösemittels geschehen, welches nachher wieder entfernt wird. Es ist aber auch möglich, die weiteren Komponenten beim Herstellprozess zuzusetzen (siehe oben). Insbesondere können die weiteren Komponenten in Schritt a) des erfindungsgemäßen Verfahrens der organischen Phase zugesetzt werden. Die weiteren Komponenten können dabei gelöst in der Polymermatrix oder auch durch eine ggf. labile kovalente Bindung an die Matrix angebunden vorliegen.

Die erfindungsgemäßen Silicon(meth-)acrylat-Partikel können eine oder mehrere Substanzen, insbesondere ausgewählt aus den oben genannten weiteren Komponenten beinhalten, welche aus den Partikeln freigesetzt werden können. Die Freisetzung kann dabei in den entsprechenden Anwendungen über einen längeren Zeitraum hinweg erfolgen. Die Freisetzung kann z. B. durch Diffusion oder Hydrolyse-Reaktionen und nachfolgender Diffusion erfolgen.

Als freizusetzende Substanzen können beispielsweise kosmetische Öle und Wirkstoffe, Duftstoffe, pharmazeutische Wirkstoffe, antimikrobielle Wirkstoffe, auch zum Beispiel Silber und silberhaltige Verbindungen, sowie Farb- und Konservierungsstoffe in den Partikeln vorhanden sein. Diese Substanzen können sowohl gelöst als auch eingebettet in der Silicon(meth-)acrylatmatrix als auch durch eine labile chemische Bindung an die Silicon(meth-)acrylatmatrix gebunden vorliegen.

Die erfindungsgemäßen bzw. erfindungsgemäß hergestellten Partikel können allein oder in Mischung mit weiteren Partikeln, Pigmenten und/oder weiteren üblichen Zusatzstoffen z. B. in Form von Pulvern oder Dispersionen in kosmetischen oder pharmazeutischen Zubereitungen oder in Pflegemitteln verwendet werden.
Die Herstellung der Dispersionen mit den erfindungsgemäßen Partikel kann mittels der üblichen Methoden entsprechend dem Stand der Technik erfolgen, jedoch ist es vorteilhaft, die nach der Polymerisation gemäß Schritt b) des erfindungsgemäßen Verfahrens und Waschen mit Alkohol/-en oder Wasser entstandenen Partikel ohne vorheriges Trocknen zu beispielsweise wässrigen Dispersionen weiter zu verarbeiten. Dies ist auch möglich, wenn beispielsweise eine gewünschte Oberflächenmodifikation direkt aus wässriger oder alkoholischer Phase erfolgen kann, was sich günstig auf die Verfahrensökonomie auswirkt.

Die erfindungsgemäßen Silicon(meth-)acrylat-Partikel bzw. diese enthaltende Dispersionen können Verwendung finden als Zusatzstoffe in Kosmetika und Toilettenartikeln, z. B. als Mattierungsmittel, als Absorber für Hautfette oder zur Erzeugung eines angenehmen, samtig-seidigen Hautgefühls, als mildes Abrasivum in Wasch- und Pflegeformulierungen sowie als über einen längeren Zeitraum hinweg Wirkstoffe oder Hilfsstoffe freisetzende Formulierungbestandteile bzw. Trägermaterialien.

Vorzugsweise ist die erfindungsgemäße Zusammensetzung eine kosmetische oder pharmazeutische Zubereitung oder ein Pflegemittel, insbesondere eine Pflegemittel für Haut und/oder Haare, bevorzugt menschliche Haut und/oder Haare.

Noch ein Gegenstand der vorliegenden Erfindung sind somit auch Pflege- und Reinigungsformulierungen, insbesondere für Haut und Hautanhangsgebilden, sowie für Haushalt und Industrie, insbesondere kosmetische Formulierungen, wobei diese bevorzugt ausgewählt sind aus der Gruppe der Haut- und Haarbehandlungsmittel, beispielsweise Formulierungen zur Hautpflege oder dekorativen Kosmetik, Shampoos mit oder ohne ausgeprägter Konditionierwirkung, Flüssigseifen und Duschgels, enthaltend erfindungsgemäße Partikel.

Eine erfindungsgemäß bevorzugte Formulierung enthält die erfindungsgemäßen Partikel in einer Menge von 0,1 Gew.-% bis 99 Gew.-%, bevorzugt in einer Menge von 0,5 Gew.-% bis 50 Gew.-%, besonders bevorzugt in einer Menge von 1,0 Gew.-% bis 20 Gew.-%, wobei sich die Gew.-% auf die Gesamtformulierung beziehen.

Erfindungsgemäße kosmetische Pflege- und Reinigungsformulierungen, können zum Beispiel mindestens eine zusätzliche Komponente enthalten, ausgewählt aus der Gruppe der
Tenside,
Emollients,
Emulgatoren,
Co-Emulgatoren
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel,
UV-Filter,
Elektrolyte,
multifunktionelle Additive,
feuchtigkeitsspendende Stoffe.

Substanzen, die als beispielhafte Vertreter der einzelnen Gruppen eingesetzt werden können, sind dem Fachmann bekannt und können beispielsweise der EP2273966A1 entnommen werden.

Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Bevorzugte erfindungsgemäße Formulierungen sind wässrige, tensidische Formulierungen; solche enthalten vorzugsweise mindestens 50 Gew.-%, bevorzugt 70 Gew.-% Wasser und mindestens ein Tensid.

In den erfindungsgemäßen Formulierungen sind neben den erfindungsgemäßen Partikeln insbesondere nichtionische Tenside der Komponente ausgewählt aus der Gruppe bestehend aus Glycerinmono- und -diester und Sorbitanmono- und -diester von gesättigten und ungesättigten Fettsäuren, Alkylmono- und -oligoglycoside, Partialester auf Basis linearer, verzweigter, ungesättigter bzw. gesättigter Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid, Cocoglucosid) sowie Polyglucoside (z.B. Cellulose), Mono-, Di- und Trialkylphosphate und deren Salze, Zitronensäureester wie z.B. Glyceryl Stearate Citrate, Glyceryl Oleate Citrate und Dilauryl Citrate sowie Glyceryl Caprylate, Polyglycerylcaprylate, Polyglycerylcaprate und Mischungen dieser Tenside enthalten.

Bevorzugte erfindungsgemäße Reinigungs- und Pflegeformulierungen für Haushalt und Industrie sind textilweichmachende Formulierungen und textilpflegende Wasch- oder Reinigungsmittel, Geschirrspülmittel, Haushaltsreiniger, Desinfektionsmittel, Desinfektionsreiniger, Schaumreiniger, Fußbodenreiniger, Teppichreiniger, Polsterreiniger, Fußbodenpflegeprodukte, Marmorreiniger, Parkettreiniger, Stein- und Keramikbodenreiniger, Wischpflegemittel, Edelstahlreiniger, Glasreiniger, Kunststoffreiniger, Sanitärreiniger, Holzreiniger, Lederreiniger, Waschmittel, Wäschepflegemittel Desinfektionswaschmittel, Vollwaschmittel, Feinwaschmittel, Wollwaschmittel, Weichspülmittel und Imprägniermittel, wobei Geschirrspülmittel und Haushaltsreinigungsmitteln, insbesondere Handgeschirrspülmittel besonders bevorzugt sind.

Besonders bevorzugte erfindungsgemäße Reinigungs- und Pflegeformulierungen für Haushalt und Industrie enthalten zusätzlich einen oder mehrere Stoffe aus der Gruppe der Tenside, Gerüststoffe, Bleichmittel, Bleichaktivatoren, Enzyme, Parfüme, Parfümträger, Fluoreszenzmittel, Farbstoffe, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber.

Insbesondere können die erfindungsgemäßen Reinigungs- und Pflegeformulierungen für Haushalt und Industrie zwischen 0,001 und 90 Gew.-%, besonders bevorzugt 0,01 bis 45 Gew.-% eines oder mehrerer der hier genannten weiteren Inhaltsstoffe enthalten, wobei sich die Gew.-% auf die gesamte Formulierung beziehen.

Beispiele für einsetzbare Tenside werden in der WO 2007/115872, Seite 17, Zeile 28 bis Seite 21, Zeile 24 beschrieben.

Beispiele für Gerüststoffe, Builder, Bleichmittel, Bleichaktivatoren, Bleichkatalysatoren und Enzyme werden in der WO 2007/115872, Seite 22, Zeile 7 bis Seite 25, Zeile 26 beschrieben.

Antiredepositionsmittel, optische Aufheller, Vergrauungsinhibitoren, Farbübertragungsinhibitoren werden beispielhaft in der WO 2007/115872 auf Seite 26, Zeile 15 bis Seite 28, Zeile 2 beschrieben.

Beispiele von Knitterschutzmitteln, antimikrobiellen Wirkstoffen, Germiziden, Fungiziden, Antioxidantien, Konservierungsmitteln, Antistatika, Bügelhilfsmitteln, UV-Absorbern werden in der WO 2007/115872 auf den Seiten 28, Zeile 14 bis Seite 30, Zeile 22 beschrieben.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

**Vergleichsbespiel 1:** Partikel aus Siliconmethacrylat mit einem mittleren molaren Verhältnis von Methacrylatgruppen zu Si Atomen von 0,055; mit Redox-Radikalstarter

163,25 g demineralisiertes Wasser und 62,50 g IDISIL SI-5530 wurden vermischt und mit Salzsäure auf pH 7 eingestellt. Das Gemisch wurde in einem Becherglas vorgelegt und unter Rühren wurden 125 g eines Siliconmethacrylats der Formel (I) mit a = 170, b = 0, c = 8, R¹ = CH₃, R³ = R' = -C₆H₁₂-O-C(O)-C(CH₃)=CH₂ zugesetzt. Das Gemisch wurde unter Verwendung eines Dispermaten bei 5000 Upm für 30 Minunten geschert. Anschließend wurden langsam 2,25 g einer 5 Gew.-%igen VARISOFT® PATC-Lösung zugetropft und das Gemisch für weitere 30 Minuten bei 5000 Upm geschert. Die stabilisierte Voremulsion wurde mit 45 g Dikaliumhydrogenphosphat gelöst in 27,0 g demineralisiertem Wasser versetzt und erneut bei 5000 Upm für 30 Minunten geschert. Die erhaltene Emulsion wurde durch Durchleiten durch einen Homogenisator (in den Beispielen wurde jeweils ein Mikrofluidizer der Firma Microfluidics eingesetzt.) mit einer Interaktionskammer mit 200 µm Durchmesser bei 800 bar Druck homogenisiert.

Zur Polymerisation wurden 325 g der Emulsion in einen 1 l Rundkolben gegeben und unter Stickstoffatmosphäre auf 80°C erwärmt. Zu der aufgeheizten Lösung wurde unter ständigem Rühren eine Lösung aus 12,5 g Ammoniumperoxodisulfat in 40 ml demineralisiertem Wasser langsam zugetropft. Anschließend wurden 7,5 g einer 39 Gew.-%igen Natriumhydrogensulfitlösung zugegeben. Die Reaktionsmischung wurde weitere 2 h gerührt bei 80 °C. Nach der Polymerisation konnte eine deutliche Braunfärbung des Reaktionsgemischs beobachtet werden. Zum Bleichen der ausgehärteten Dispersion wurden bei 80 °C 15 g einer 30%igen Wasserstoffperoxid unter ständigem Rühren zugegeben, wobei eine deutlich Aufhellung des Reaktionsgemisch festgestellt werden konnte. Anschließend wurde die Partikeldispersion langsam auf Raumtemperatur abgekühlt und filtriert. Die erhaltenen Partikel wurden fünfmal mit je 150 ml Wasser gewaschen und in einem Vakuumtrockenschrank bei 50 °C bis zur Massenkonstanz getrocknet. Durch eine elektronenmikroskopische Betrachtung der Partikel konnte eine mittlere Größe der Partikel von ungefähr 10 µm nachgewiesen werden. Hierbei war auffällig, dass die Partikel Einfallstellen ("Dimpel") aufwiesen.

Zur Bestimmung des Anteils an nicht abreagierten aber kovalent an die Partikel gebundenen Methacrylat-Gruppen wurden diese durch alkalische Esterspaltung hydrolysiert und die hierbei freigesetzte Methacrylsäure mittels HPLC quantifiziert. Hierfür wurden 0,5 g der Partikel in 25 ml 1 normaler ethanolischer Kalilauge, 5 ml H₂O und 1 ml Diethylenglycolmonoethylether für eine Stunde im Rückfluss gekocht. Das Hydrolysat wurde mit wenig Ethanol in einen 50 ml Messkolben überführt und aufgefüllt. Anschließend wurde die Probe im Verhältnis 1:10 mit Wasser verdünnt und dabei mit konzentrierter Schwefelsäure auf pH 2 eingestellt. Die anschließende chromatographische Trennung der Probe erfolgte mittels RP HPLC mit UV-Detektion (HPLC-System: Agilent 1100, Säule: Macchery & Nagel Pyramid C18). Hierbei konnte ein Restmethacrylatgehalt von 11000 ppm ermittelt werden.

### Erfindungsgemäßes Beispiel 1:

Partikel aus Siliconmethacrylat mit einem mittleren molaren Verhältnis von Methacrylatgruppen zu Si Atomen von 0,055; mit öllöslichem Radikalstarter

228,5 g demineralisiertes Wasser und 50,0 g IDISIL SI 5530 wurden vermischt und mit Salzsäure auf pH 7 eingestellt. Das Gemisch wurde in einem Becherglas vorgelegt und unter Rühren wurden 100 g eines Siliconmethacrylats der Formel (I) mit a = 170, b = 0, c = 8, R¹ = CH₃, R³ = R' = -C₆H₁₂-O-C(O)-C(CH₃)=CH₂, in welchem zuvor 0,3 g Laurylperoxid gelöst wurden, zugefügt. Anschließend wurde das Gemisch unter Verwendung eines Dispermaten bei 5000 Upm für 30 Minuten geschert. Anschließend wurden langsam 5,5 g einer 1 Gew.-%igen VARISOFT® PATC-Lösung zugetropft und das Gemisch für weitere 30 Minuten bei 5000 Upm geschert. Die erhaltene Voremulsion wurde durch Durchleiten durch einen Homogenisator mit einer Interaktionskammer mit 200 µm Durchmesser bei 800 bar Druck homogenisiert.

Zur Polymerisation wurde die Emulsion in einen 500 ml Rundkolben überführt und anschließend unter Rühren 45 min lang ein Stickstoffstrom eingeleitet. Die Reaktionsmischung wurde anschließend für auf 80°C erwärmt und für 3 h unter gelindem Rühren bei dieser Temperatur gehalten. Nach Abkühlen wurde die so ausgehärtete Partikeldispersion filtriert. Die erhaltenen Partikel wurden zweimal mit Wasser gewaschen und anschließend in einem Vakuumtrockenschrank bei 50 °C bis zur Massenkonstanz getrocknet. Durch eine elektronenmikroskopische Betrachtung der Partikel konnte eine mittlere Größe der Partikel von ungefähr 10 µm nachgewiesen werden. Im Gegensatz zu den in Vergleichsbeispiel V1 beschriebenen Partikeln hatten die Partikel eine "kugeligere" Morphologie und es konnten keine Einfallstellen auf den Partikeln nachgewiesen werden.

Zur Bestimmung des Anteils an nicht abreagierten aber kovalent an die Partikel gebundenen Methacrylat-Gruppen wurde das in Vergleichsbeipiel V1 beschriebene Analytik-Verfahren verwendet. Hierbei konnte ein Restmethacrylatgehalt von 1040 ppm nachgewiesen werden. Dieser liegt somit um mehr als einen Faktor zehn unter dem nachgewiesenen Restmethacrylatgehalt der in Vergleichsbeispiel 1 beschriebenen Partikel.

### Vergleichsbespiel V1:

Partikel aus Siliconmethacrylat mit einem mittleren molaren Verhältnis von Methacrylatgruppen zu Si Atomen von 0,25; mit öllöslichem Radikalstarter

228,5 g demineralisiertes Wasser und 50,0 g IDISIL SI 5530 wurden vermischt und mit Salzsäure auf pH 7 eingestellt. Das Gemisch wurde in einem Becherglas vorgelegt und unter Rühren wurden 100 g eines Siliconmethacrylats der Formel (I) mit a = 15, b = 0, c = 5, R¹ = R³ = CH₃, R' = (CH₂)₃-O-CH₂-CH(OH)-CH₂-O-C(O)-C(CH₃)=CH₂, in welchem zuvor 0,3 g Laurylperoxid gelöst wurden, zugefügt. Anschließend wurde das Gemisch unter Verwendung eines Dispermaten bei 5000 Upm für 30 Minuten geschert. Anschließend wurden langsam 5,5 g einer 1 Gew.-%igen VARISOFT® PATC-Lösung zugetropft und das Gemisch für weitere 30 Minuten bei 5000 Upm geschert. Die erhaltene Voremulsion wurde durch Durchleiten durch einen Homogenisator mit einer Interaktionskammer mit 200 µm Durchmesser bei 800 bar Druck homogenisiert.

Zur Polymerisation wurde die Emulsion in einen 500 ml Rundkolben überführt und anschließend unter Rühren 45 min lang ein Stickstoffstrom eingeleitet. Die Reaktionsmischung wurde anschließend auf 80°C erwärmt und für 3 h unter gelindem Rühren bei dieser Temperatur gehalten. Nach Abkühlen wurde die so ausgehärtete Partikeldispersion filtriert. Die erhaltenen Partikel wurden zweimal mit Wasser gewaschen und anschließend in einem Vakuumtrockenschrank bei 50 °C bis zur Massenkonstanz getrocknet.

### Erfindungsgemäßes Beispiel 2:

Partikel aus Siliconmethacrylat mit einem mittleren molaren Verhältnis von Methacrylatgruppen zu Si Atomen von 0,047; mit öllöslichem Radikalstarter

228,5 g demineralisiertes Wasser und 50,0 g IDISIL SI 5530 wurden vermischt und mit Salzsäure auf pH 7 eingestellt. Das Gemisch wurde in einem Becherglas vorgelegt und unter Rühren wurden 100 g eines Siliconmethacrylats der Formel (I) mit a = 83, b = 0, c = 0, R¹ = CH₃, R³ = (CH₂)₃-O-CH₂-C(CH₂-O-C(O)-C(CH₃)=CH₂)₂-CH₂-CH₃, in welchem zuvor 0,3 g Laurylperoxid gelöst wurden, zugefügt. Anschließend wurde das Gemisch unter Verwendung eines Dispermaten bei 5000 Upm für 30 Minuten geschert. Anschließend wurden langsam 5,5 g einer 1 Gew.-%igen VARISOFT® PATC-Lösung zugetropft und das Gemisch für weitere 30 Minuten bei 5000 Upm geschert. Die erhaltene Voremulsion wurde durch Durchleiten durch einen Homogenisator mit einer Interaktionskammer mit 200 µm Durchmesser bei 800 bar Druck homogenisiert.

Zur Polymerisation wurde die Emulsion in einen 500 ml Rundkolben überführt und anschließend unter Rühren 45 min lang ein Stickstoffstrom eingeleitet. Die Reaktionsmischung wurde anschließend für auf 80°C erwärmt und für 3 h unter gelindem Rühren bei dieser Temperatur gehalten. Nach Abkühlen wurde die so ausgehärtete Partikeldispersion filtriert. Die erhaltenen Partikel wurden zweimal mit Wasser gewaschen und anschließend in einem Vakuumtrockenschrank bei 50 °C bis zur Massenkonstanz getrocknet.

Die nachfolgend aufgeführten Formulierungsbeispiele sind in der Mehrzahl Emulsionen vom Typ Öl-in-Wasser (O/W) oder Wasser-in-ÖI (W/O). Diese können nach üblichen, dem Fachmann bekannten, Methoden unter Verwendung typischer Rühraggregate hergestellt werden. Vorzugsweise werden W/O-Emulsionen durch langsames Einrühren der Wasserphase in die Ölphase mit nachfolgender Homogenisierung hergestellt. Bei den beschriebenen O/W Emulsionen werden vorzugsweise Öl- und Wasserphase ohne Rühren zusammengeführt und anschließend homogenisiert. Dies kann in einem Kalt-Kalt-Prozess geschehen oder in einem Heiss-Heiss-Prozess, bei dem die Homogenisierung bei ca. 70 °C stattfindet. Die erfindungsgemäßen Silicon(meth)acrylatpartikel können dabei prinzipiell in jeder Stufe des Prozesses eingearbeitet werden. In den meisten Beispielformulierungen erfolgte entweder die Einarbeitung in die fertige Emulsion bei Temperaturen < 60 °C oder die Silicon(meth)acrylatpartikel wurden direkt zu Beginn der Emulsionsherstellung zusammen mit der Ölphase vorgelegt.

Der zur Auswertung der Emulsionsvergleichsbeispiele verwendeten Nomenklatur zum Thema "Einarbeitbarkeit" liegen folgende Anforderungen zugrunde. Unter gleichen Bedingungen was Zeitpunkt der Zugabe der Silicon(meth)acrylatpartikel und Nutzung von Scheraggregaten und -geschwindigkeiten betrifft, beschreibt eine leichte Einarbeitbarkeit keinen unüblichen Aufwand bei der Herstellung der Formulierung und das Ergebnis ist eine gleichmäßige Verteilung der Silicon(meth)acrylatpartikel ohne störende Agglomerate. Ist die Einarbeitung schwierig, ist bei Verwendung eines gleichen Scheraggregats und der gleichen Schergeschwindigkeit, ein längerer Schereintrag zu erbringen, um eine agglomeratfreie Formulierung zu erlangen, oder Partikelagglomerate lassen sich nicht ohne die Sensorik störende Agglomerate einarbeiten.

Der zur Auswertung der Emulsionsvergleichsbeispiele verwendeten Nomenklatur zum Thema "Stabilität" liegen folgende Anforderungen zugrunde. Ist die Stabilität mit "gut" bewertet, so bedeutet das, dass eine solche Emulsion mindestens einen Monat bei Raumtemperatur, -5 °C und 40 °C stabil ist. "Stabil" bedeutet dabei, dass keinerlei Öl- oder Wasserabscheidung auftritt, dass das Erscheinungsbild der Emulsion homogen bleibt und dass in der Emulsion keine nennenswerten Veränderungen von Viskosität, Farbe oder Geruch auftreten.

Das Hautgefühl der in den folgenden Beispielen beschriebenen kosmetischen Formulierungen wurde durch ein sogenanntes Panel bestimmt. Mindestens fünf Personen verglichen die sensorischen Eigenschaften der kosmetischen Formulierungen und der jeweiligen Vergleichsformulierung ohne die Zusammensetzung zu kennen. Es werden die Eigenschaften aufgeführt, die die Mehrheit der Personen bevorzugt beschrieben hat.

### Beispiel 3 und Vergleichsbeispiel V3:

Es wurden die in Tabelle 1a angegebenen Formulierungen hergestellt. Die Bewertung der Formulierungen erfolgte in Bezug auf deren Einarbeitbarkeit, Stabilität, Erscheinungsbild und Hautgefühl und ist in Tabelle 1b angegebenen.

**Tabelle 1a: Formulierungen zu Beispiel 3 und Vergleichsbeispiel V3, Öl-in-Wasser Pflegecreme**

| | **Beispiel** | **3** | **V3** |
|---|---|---|---|
| **A** | TEGO® Care 165 (Evonik Industries AG) (Glycerylstearat; PEG-100 Stearat) | 6,0 % | 6,0 % |
| | Stearylalkohol | 3,0 % | 3,0 % |
| | Mineralöl | 4,0 % | 4,0 % |
| | Ethylhexylpalmitat | 4,0 % | 4,0 % |
| **B** | Glycerin | 3,0 % | 3,0 % |
| | Wasser | 75,0 % | 75,0 % |
| **C** | Siliconmethacrylat-Partikel aus erfindungsgemäßen Bsp. 1 | 5,0 % | |
| | Siliconmethacrylat-Partikel aus Vergleichsbsp. V1 | | 5.0 % |
| **Z** | Methylparaben, Ethylparaben, Methylisothiazolinon, Parfum | q.a. | q.a. |

**Tabelle 1b: Ergebnisse von Beispiel 3 und Vergleichsbeispiel V3, Öl-in-Wasser Pflegecreme**

| | **Beispiel** | **3** | **V3** |
|---|---|---|---|
| | Einarbeitbarkeit | Leicht, Agglomerate sind gut durch Scherung aufzutrennen | Schwieriger als bei Beispiel 3, Auftrennung der Agglomerate dauert länger |
| | Stabilität | Gut | Gut |
| | Erscheinungsbild | Weiß, homogen | Weiß, homogen |
| | Hautgefühl | Während der Anwendung: Samtig-seidig, sehr glatt, nicht stumpf; 5 Minuten nach Anwendung: sehr glatt, pudrig | Während der Anwendung: Samtig-seidig, glatt; 5 Minuten nach Anwendung: etwas stumpf, trocken |

### Beispiel 4 und Vergleichsbeispiel V4: Wasser-in-ÖI Foundation

Es wurden die in Tabelle 2a angegebenen Formulierungen hergestellt. Die Bewertung der Formulierungen erfolgte in Bezug auf deren Stabilität, Erscheinungsbild und Hautgefühl und ist in Tabelle 2b angegeben.

**Tabelle 2a: Wasser-in-ÖI Foundation gemäß Beispiel 4a, 4b und Vergleichsbeispiel V4:**

| | **Beispiel** | **4a** | **4b** | **V4** |
|---|---|---|---|---|
| A | ABIL® EM 90 (Evonik Industries AG) (Cetyl PEG/PPG-10/1 Dimethicone) | 3,0 % | 3,0 % | 3,0 % |
| | Diethylhexylcarbonat | 10,0 % | 10,0 % | 10,0 % |
| | Cyclopentasiloxan | 7,6 % | 7,6 % | 7,6 % |
| | Ethylhexylpalmitat | 3,4 % | 3,4 % | 3,4 % |
| | Eisenoxid | 1,8 % | 1,8 % | 1,8 % |
| | Titandioxid | 7,2 % | 7,2 % | 7,2 % |
| | Talkum | 2,0 % | 2,0 % | 2,0 % |
| | Siliconmethacrylat-Partikel aus erfindungsgemäßem Bsp. 1 | 2,5 % | | |
| | Siliconmethacrylat-Partikel aus erfindungsgemäßem Bsp. 2 | | 2,5% | |
| | Siliconmethacrylat-Partikel aus Vergleichsbsp. 2 | | | 2,5% |
| B | NaCl | 1,0 % | 1,0 % | 1,0 % |
| | Glycerin | 2,0 % | 2,0 % | 2,0 % |
| | Wasser | 59,5 % | 59,5 % | 59,5 % |
| Z | Phenoxyethanol; Methylparaben; Ethylparaben, Butylparaben; Propylparaben, Isobutylparaben, Parfum | q.a. | q.a. | q.a. |

**Tabelle 2b: Wasser-in-ÖI Foundation gemäß Beispiel 4a, 4b und Vergleichsbeispiel V4:**

| | **Beispiel** | **4a** | **4b** | **V4** |
|---|---|---|---|---|
| | Einarbeitbarkeit | Leicht, Agglomerate sind gut durch Scherung aufzutrennen | Leicht, Agglomerate sind gut durch Scherung aufzutrennen | Leicht, Agglomerate sind gut durch Scherung aufzutrennen |
| | Stabilität | Gut | Gut | Gut |
| | Erscheinungsbild | Bräunlich, homogen | Bräunlich, homogen | Bräunlich, homogen |
| | Hautgefühl | Während der Anwendung: Samtig-seidig, pudrig sehr glatt, nicht stumpf; 5 Minuten nach Anwendung: sehr glatt, samtig-seidig | Während der Anwendung: Samtig-seidig, pudrig glatt, nicht stumpf; 5 Minuten nach Anwendung: glatt, samtig-seitig | Während der Anwendung: pudrig, stumpf; 5 Minuten nach Anwendung: stumpf, trocken |

### Weitere Formulierungsbeispiele:

### Beispiel 5: O/W Serum mit kosmetischen Wirkstoffen gemäß Tabelle 3

**Tabelle 3: O/W Serum mit kosmetischen Wirkstoffen gemäß Bespiel 5**

| | |
|---|---|
| Polyglyceryl-6 Stearate (and) Polyglyceryl-6 Behenate* | 2,5% |
| Caprylic/Capric Triglyceride** | 2,0% |
| Oleyl Erucate *** | 2,0% |
| Persea Gratissima (Avocado) Oil | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate**** | 3,0% |
| Wasser | ad 100% |
| Butylenglycol | 5,0% |
| Tetrapeptide-21; Glycerin; Butylene Glycol; Aqua***** | 2,0% |
| Hydrolyzed Hyaluronic Acid****** | 0,1% |
| Siliconmethacrylat-Partikel aus Bsp. 1 | 1,00% |
| Xanthan Gum | 0,5% |
| Sodium Hydroxide (10% aq.) | 0,2% |
| Benzyl Alcohol, Benzoic Acid, Sorbic Acid | 0,8% |
| Polyglutamic acid; Sclerotium Glucan; Betaine; Urea; Potassium Lactate******* | 3,0% |

| | |
|---|---|
| *TEGO® Care PBS 6 (Evonik Industries AG) **TEGOSOFT® CT (Evonik Industries AG) ***TEGOSOFT® OER (Evonik Industries AG) ****Hyacare® Filler CL (Evonik Industries AG) *****TEGO® PEP 4-17 (Evonik Industries AG) ******HyaCare® 50 (Evonik Industries AG) *******TEGO® Smooth Complex (Evonik Industries AG) | |

### Beispiel 6: O/W Creme gemäß Tabelle 4

**Tabelle 4: O/W Creme gemäß Beispiel 6**

| | |
|---|---|
| Polyglyceryl-3-Methylglucosedistearat | 3,00% |
| Glycerylstearat | 2,00% |
| Cetearylalkohol | 1,00% |
| Ethylhexylstearat | 10,00% |
| Decyloleat | 9,00% |
| Siliconmethacrylat-Partikel aus Bsp. 1 | 1,50% |
| Glycerin | 3,00% |
| Wasser | ad 100% |
| Natriumbenzoat, Kaliumsorbat, Phenoxyethanol, Parfum | q.s. |

### Beispiel 7: Tränklösung für Feuchttücher gemäß Tabelle 5

**Tabelle 5: Tränklösung für Feuchttücher gemäß Beispiel 7**

| | |
|---|---|
| Ethylhexylstearat, Phenoxyethanol, Sorbitanlaurat, Polyglyceryl-4-laurat, Dilaurylcitrat* | 5,70% |
| Cyclomethicone | 2,00% |
| Siliconmethacrylat-Partikel aus Bsp. 1 | 2,00% |
| Wasser | ad 100% |
| Glycerin | 3,00% |
| Carbomer** | 0,10%. |
| Natriumhydroxid (10% in Wasser) | q.s. |

| | |
|---|---|
| *TEGO® Wipe Flex (Evonik Industries AG) **TEGO® Carbomer 141 (Evonik Industries AG) | |

Die Tränklösung kann mit Hilfe üblicher Tränk- oder Sprühprozesse zur Herstellung von kosmetischen Feuchttüchern verwendet werden (z.B. für Babypflege, Make-Up Entferner, Reinigungstücher). Dazu werden typischerweise Vliesstoffe (non-woven) verwendet, die in der Regel Fasern von Polyolefinen, Polyestern, Cellulosen, Rayon, Polyamiden, Polyesteramiden oder Polyvinylalkoholen aufweisen oder aus diesen bestehen oder die aus gemischten Fasern dieser Komponenten aufgebaut sind.

### Beispiel 8: Make-Up Powder Foundation gemäß Tabelle 6

**Tabelle 6: Make-Up Powder Foundation gemäß Beispiel 8:**

| | |
|---|---|
| Zinkstearat | 3,00% |
| Glimmer | 40,00% |
| Talkum | 24,00% |
| Eisenoxid | 5,00% |
| Siliconmethacrylat-Partikel aus Bsp. 1 | 10,00% |
| Titandioxid | 8,00% |
| Cetylethylhexanoat | 2,00% |
| Squalan | 3,00% |
| Cetearylethylhexanoat | 2,00%. |
| Mineralöl (30 mPas) | 2,00% |
| PEG/PPG-4/12 Dimethicone | 1,00% |
| Aluminium Stärke Octenylsuccinat | q.s. |
| Eisenoxid | q.s. |
| Parfum | q.s. |

### Beispiel 9: Make-Up Foundation gemäß Tabelle 7

**Tabelle 7: Make-Up Foundation gemäß Beispiel 9**

| | |
|---|---|
| Phenyltrimethicone | 14,00% |
| Ethylhexylpalmitat | 14,60% |
| Cetylethylhexanoat | 5,00% |
| Carnaubawachs | 4,70% |
| Stearoxydimethicone | 4,00% |
| PVP/Eicosene Copolymer | 1,00% |
| Cetylstearylheptanoat | 2,85% |
| Covabead LH 85, Polymethylmethacrylat Partikel | 3,00% |
| Siliciumdioxid | 0,25%. |
| Zinkoxid | 7,00% |
| Nylon-12 | 2,00% |
| Talc Covasil 4.05 | 9,50% |
| Acrylat Copolymer | 2,00% |
| Siliconmethacrylat-Partikel aus Bsp. 1 | 3,00% |
| Aluminium Stärke Octenylsuccinat | 9,50% |
| Eisenoxid | 3,10% |
| Titandioxid (und) Dimethicone | 14,50% |

### Beispiel 10 Lidschattenformulierung gemäß Tabelle 8

**Tabelle 8: Lidschattenformulierung gemäß Beispiel 10**

| | |
|---|---|
| Cyclomethicone | ad 100% |
| PPG-3 Myristylether | 7,00% |
| Polyglyceryl-4-Isostearat; Cetyl PEG/PPG-10/1 Dimethicone; Hexyllaurat* | 1,00% |
| Dimethicone (20 mPas) | 2,50% |
| Cera Alba | 4,50% |
| Carnaubawachs | 2,00% |
| A-C Coploymer 400 (Ethylen/VA Copolymer) | 2,50% |
| Ozokerit | 5,80% |
| C18-36-Säuretriglycerid | 2,00% |
| Liquiparöl (Isobutylparaben (und) Isopropylparaben (und) Butylparaben) | 0,20% |
| Siliconmethacrylat-Partikel aus Bsp. 1 | 4,00% |
| Titandioxid | 5,00% |
| Chromoxid Grün) | 10,00% |
| CI 77491 (und) Aluminium Puder (und) Siliciumdioxid | 5,00% |
| CI 77891 (und) CI 77288 (und) Glimmer | 10,00% |

| | |
|---|---|
| *ABIL® WE 09 (Evonik Industries AG) | |

### Beispiel 11: O/W Sonnenschutzlotion gemäß Tabelle 9

**Tabelle 9: O/W Sonnenschutzlotion gemäß Beispiel 11**

| | |
|---|---|
| Glycerylstearatcitrat | 3,00% |
| Cetearylalkohol | 1,00% |
| Cetyldimethicone | 0,20% |
| C₁₂-C₁₅ Alkyl Benzoat | 4,80% |
| Triisostearin | 1,00% |
| Diethylhexylcarbonat | 6,00% |
| Titandioxid; Trimethoxycaprylylsilan* | 3,00% |
| Tocopherylacetat | 0,50% |
| Ethylhexylmethoxycinnamat | 5,00%. |
| Butylmethoxydibenzoylmethan | 2,50% |
| Carbomer | 0,20% |
| Xanthan | 0,40% |
| Natriumcarboxymethylbetaglucan | 0,10% |
| Glycerin | 2,00% |
| Wasser | ad 100% |
| Siliconmethacrylat-Partikel aus Bsp. 1 | 1.50% |
| Natriumhydroxid (10% in Wasser) | q.s. |
| Parfum | q.s. |

| | |
|---|---|
| *Tego® Sun T 805 (Evonik Industries AG) | |

**Beispiel 12 AP/Deo Roll-On gemäß Tabelle 10:**

**Tabelle 10: Formulierung gemäß Beispiel 12**

| | |
|---|---|
| Steareth-2 | 2,20% |
| Steareth-20 | 1,00% |
| Cetearylethylhexanoat | 2,00% |
| PPG-11 Stearylether | 2,00% |
| Dimethicone | 0,50% |
| Polyglyceryl 3-Caprylat | 0,50% |
| Aluminiumchlorohydrat | 5,00% |
| Wasser | ad 100% |
| Glycerin | 3,00% |
| Siliconmethacrylat-Partikel aus Bsp. 1 | 0,30%. |
| Parfum | q.s. |
| Citronensäure (50% in Wasser) | q.s. |
| Phenoxyethanol, Ethylhexylglycerin | q.s. |

### Beispiel 13 Lippenstiftformulierung gemäß Tabelle 11:

**Tabelle 11: Formulierung gemäß Beispiel 13**

| | |
|---|---|
| Cyclopentasiloxan | 34,00% |
| Behenoxydimethicone | 3,00% |
| Stearyldimethicone | 10,00% |
| Polyisobuten | 5,00% |
| Phenyltrimethicone | 8,00% |
| Isododecan | 4,00% |
| Bis-Diglyceryl Polyacyladipat-2 | 4,00% |
| Ceresin | 24,00% |
| Titandioxid | 1,00% |
| Karminrot | 1,00% |
| D&C Red Nr. 7 | 3,00% |
| Polyethylen | 1,00% |
| Siliconmethacrylat-Partikel aus Bsp. 1 | 1,00% |
| Aluminium Stärke Octenylsuccinat & Lauroyllysin | 1,00% |

### Beispiel 14 Mascaraformulierung gemäß Tabelle 12:

**Tabelle 12: Formulierung gemäß Beispiel 14**

| | |
|---|---|
| Sucrosestearat | 4,00% |
| Polyglyceryl-3 Methylglucosedistearat | 2,00% |
| Stearylalkohol | 1,00% |
| Cadelillawachs | 5,00% |
| Carnaubawachs | 1,75% |
| Bienenwachs | 4,25% |
| Hydriertes Reiskleiewachs | 5,00% |
| Adipinsäure/Diethylenglycol/Glycerin Crosspolymer | 5,00% |
| Ceramid NP | 0,05% |
| Eisenoxid | 10,00% |
| Siliconmethacrylat-Partikel aus Bsp. 1 | 0,50% |
| Wasser | ad 100% |
| 1,3-Butandiol | 3,00% |
| Triethanolamin | 1,80% |
| Acrylate/Octylacrylamid Copolymer | 5,00% |
| Phenoxyethanol; Methylparaben; Ethylparaben, Butylparaben; Propylparaben, Isobutylparaben | 0,60% |
| Phenoxyethanol | 0,50% |

### Beispiel 15 Make-up Foundation gemäß Tabelle 13:

**Tabelle 13: Formulierung gemäß Beispiel 15**

| | |
|---|---|
| Bis-(Glyceryl/Lauryl) Glyceryl/Lauryl Dimethicone* | 4,00% |
| Diethyl hexylcarbonat** | 3,10% |
| Phenoxyethylcaprylat*** | 3,10% |
| Mineralöl | 6,30% |
| Titandioxid | 4,00% |
| Eisenoxid | 2,50% |
| Talkum | 1,00% |
| Bornitrid | 1,00% |
| Dicaprylylcarbonat (und) Stearalkonium Hectorit (und) Propylen carbonate | 2,00% |
| Siliconmethacrylat-Partikel aus Bsp. 1 | 1,00% |
| Nylon-10/10**** | 1,00% |
| Dimethicone; Dimethicone/Vinyl Dimethicone Crosspolymer | 4,00% |
| Glycerin | 4,00% |
| Magnesiumsulfat Heptahydrat | 1,50% |
| Wasser | ad 100% |
| Methylparaben; Ethylparaben; Propylparaben; n-Propylparaben; Phenoxtol | 0,70% |

| | |
|---|---|
| *ABIL® EM 120 (Evonik Industries AG) **TEGOSOFT® DEC (Evonik Industries AG) ***TEGOSOFT® XC (Evonik Industries AG) ****TEGOLON® ECO 10-10 (Evonik Industries AG) | |

### Beispiel 16 Blemish Balm mit SPF 15-Formulierung gemäß Tabelle 14:

**Tabelle 14: Blemish Balm mit SPF 15-Formulierung gemäß Beispiel 16**

| | |
|---|---|
| Polyglyceryl-3 Methylglucose Distearate* | 3,00% |
| Glyceryl Stearate | 2,50% |
| Stearyl Alcohol | 1,50% |
| Diethylhexyl Carbonate** | 6,90% |
| Aqua; Ethylhexyl Stearate; Sodium Hyaluronate Crosspolymer; Polyglyceryl-4 Diisostearate/Polyhydroxystearate/Sebacate; Sodium Isostearate*** | 2,00% |
| Phytosphingosine | 0,10% |
| Ethylhexyl Methoxycinnamate | 5,00% |
| Diethylamino Hydroxybenzoyl Hexyl Benzoate | 3,00% |
| Titanium Dioxide Cl 77891 | 3,00% |
| Talkum | 2,00% |
| Gelbes Eisenoxid**** | 0,36% |
| Rotes Eisenoxid***** | 0,12% |
| Schwarzes Eisenoxid****** | 0,08% |
| Isoamyl Cocoate******* | 4,44% |
| Phenoxyethyl Caprylate******** | 4,00% |
| Nylon-10/10********* | 1,50% |
| Siliconmethacrylat-Partikel aus Bsp. 1 | 1,50% |
| Wasser | ad 100% |
| Glycerin | 3,00% |
| Hydrolyzed Hyaloronic Acid********** | 0,10% |
| Tetrapeptide-21; Glycerin; Butylene Glycol; Aqua*********** | 2,00% |
| Ethanol | 3,00% |
| Methylparaben, Ethylparaben, Methylisothiazolinone | 0,80% |

| | |
|---|---|
| *TEGO® Care 450 (Evonik Industries AG) **TEGOSOFT® DEC (Evonik Industries AG) ***HyaCare® Filler CL (Evonik Industries AG) ****Unipure Yellow LC 182 (Sensient) *****Unipure Red LC 381 (Sensient) ******Unipure Black LC 989 (Sensient) *******TEGOSOFT® AC (Evonik Industries AG) ********TEGOSOFT® XC (Evonik Industries AG) *********TEGOLON® ECO 10-10 (Evonik Industries AG) **********HyaCare® 50 (Evonik Industries AG) ***********TEGO® Pep 4-17 (Evonik Industries AG) | |

### Beispiel 17 Duschgel-Formulierung gemäß Tabelle 15:

**Tabelle 15: Formulierung gemäß Beispiel 17**

| | |
|---|---|
| Acrylate/C10-30 Alkyl Acrylat Crosspolymer* | 1,60% |
| Wasser | ad 100% |
| Natriumlaurylsulfat | 21,40% |
| Cocamidopropylbetain** | 5,30% |
| Polyglyceryl-3 Caprat*** | 0,50% |
| Siliconmethacrylat-Partikel aus Bsp. 1 | 2,00% |
| Natriumhydroxid (10% in Wasser) | q.s. |
| Phenoxyethanol; Methylisothiazolinon | q.s. |
| Parfum | q.s. |

| | |
|---|---|
| *TEGO® Carbomer 341 ER (Evonik Industries AG) **TEGO® Betain F 50 (Evonik Industries AG) ***TEGOSOFT® PC 31 (Evonik Industries AG) | |

### Beispiel 18 Conditioner-Formulierung gemäß Tabelle 16:

**Tabelle 16: Formulierung gemäß Beispiel 18**

| | |
|---|---|
| Wasser | ad 100% |
| Cetrimoniumchlorid* | 2,00% |
| Behentrimoniumchlorid** | 2,00% |
| Cyclopentasiloxan; Dimethiconol*** | 1,00% |
| Siliconmethacrylat-Partikel aus Bsp. 1 | 0,50% |
| Cetearylalkohol**** | 5,00% |
| Phenoxyethanol; Methylisothiazolinon | q.s. |
| Parfum | q.s. |

| | |
|---|---|
| *VARISOFT® 300 (Evonik Industries AG) **VARISOFT® BT 85 (Evonik Industries AG) ***ABIL® OSW 5 (Evonik Industries AG) ****TEGO® Alkanol 1618 (Evonik Industries AG) | |

### Beispiel 19: 2-in-1-Shampoo-Formulierung gemäß Tabelle 17

**Tabelle 17: Formulierung gemäß Beispiel 19**

| | |
|---|---|
| Natriumlaurylsulfat | 60,00% |
| Natriumcumolsulfonat | 3,00% |
| Wasser | ad 100% |
| Cocamidopropylbetain* | 8,00% |
| Cocamid MEA** | 1,50% |
| Glycoldistearat*** | 1,50% |
| Cetylalkohol**** | 0,50% |
| Dimethicone (1000 mPa*s) | 1,50% |
| Siliconmethacrylat-Partikel aus Bsp. 1 | 1,00% |
| Nylon-10/10***** | 0,50% |
| Xanthan Gum | 0,75% |
| Phenoxyethanol; Methylisothiazolinon | q.s. |
| Parfum | q.s. |

| | |
|---|---|
| *TEGO® Betain F 50 (Evonik Industries AG) **REWOMID® C 212 (Evonik Industries AG) ***TEGIN® G 100 (Evonik Industries AG) ****TEGO® Alkanol 16 (Evonik Industries AG) *****TEGOLON® ECO 10-10 (Evonik Industries AG) | |

### Beispiel 20 Styling Wax-Formulierung gemäß Tabelle 18:

**Tabelle 18: Formulierung gemäß Beispiel 20**

| | |
|---|---|
| Wasser | ad 100% |
| Propylenglykol | 2,00% |
| Glycerin | 11,00% |
| Methoxy PEG/PPG-7/3 Aminopropyldimethicone* | 0,50% |
| Isosteareth-20** | 14,50% |
| Laureth-4*** | 10,00% |
| Paraffinum Perliquidum | 6,00% |
| C12-15 Alkyl Benzoate**** | 6,00% |
| Siliconmethacrylat-Partikel aus Bsp. 1 | 1,00% |
| Phenoxyethanol; Methylisothiazolinon | q.s. |
| Parfum | q.s. |

| | |
|---|---|
| *ABIL® Soft AF 100 (Evonik Industries AG) **REWODERM® 66 E 20 (Evonik Industries AG) ***TEGO® Alkanol L 4 (Evonik Industries AG) ****TEGOSOFT® TN (Evonik Industries AG) | |

### Beispiel 21 Leave-in-Conditioner-Formulierung gemäß Tabelle 19:

**Tabelle 19: Formulierung gemäß Beispiel 21**

| | |
|---|---|
| Ceteareth-25* | 4,00% |
| Cyclopentasiloxan; Dimethiconol** | 16,00% |
| Methoxy PEG/PPG-7/3 Aminopropyldimethicone*** | 1,00% |
| Siliconmethacrylat-Partikel aus Bsp. 1 | 3,00% |
| Laureth-4**** | 0,50% |
| Carbomer***** | 0,50% |
| Wasser | ad 100% |
| Propylenglykol | 5,00% |
| Natriumhydroxid (10 % in Wasser) | ad pH 5-6 |
| Parfum | q.s. |
| Phenoxyethanol; Methylisothiazolinon | q.s. |

| | |
|---|---|
| *TEGIN® ACID C (Evonik Industries AG) **ABIL® OSW 5 (Evonik Industries AG) ***ABIL® Soft AF 100 (Evonik Industries AG) ****TEGO® Alkanol L 4 (Evonik Industries AG) *****TEGO® Carbomer 140 (Evonik Industries AG) | |

### Beispiel 22 Leichte W/O Creme gemäß Tabelle 20:

**Tabelle 20: Formulierung gemäß Beispiel 22**

| | |
|---|---|
| Bis-PEG/PPG-14/14 Dimethicone; Caprylic/Capric Triglyceride* | 3,00% |
| Hydrogenated Castor Oil | 0,80% |
| Microcrystalline Wax | 1,20% |
| Isopropyl Palmitate** | 4,30% |
| Isohexadecane | 4,30% |
| Dimethicone (5 mPas) | 10,40% |
| Siliconmethacrylat-Partikel aus Bsp. 1 | 2,00% |
| Glycerin | 3,00% |
| Sodium Chloride | 2,00% |
| Creatine*** | 0,50% |
| Wasser | ad 100,00 % |
| Phenoxyethanol, Ethylhexylglycerin | 0,70% |
| Parfum | q.s. |

| | |
|---|---|
| *ABIL® EM 97 S (Evonik Industries AG) **TEGOSOFT® P (Evonik Industries AG) ***TEGO® Cosmo C100 (Evonik Industries AG) | |

### Beispiel 23 W/O Feuchtigkeitscreme gemäß Tabelle 21:

**Tabelle 21: Formulierung gemäß Beispiel 23**

| | |
|---|---|
| Cetyl PEG/PPG/10/1 Dimethicone* | 2,00% |
| Mineral Oil (30 mPas) | 17,00% |
| Hydrogenated Castor Oil | 0,40% |
| Siliconmethacrylat-Partikel aus Bsp. 1 | 2,00% |
| Microcrystalline Wax | 0,60% |
| Sodium Chloride | 0.50% |
| Wasser | Ad 100,00% |
| Urea | 10,00% |
| Phenoxyethanol, Ethylhexylglycerin | 0,70% |
| Parfum | q.s. |

| | |
|---|---|
| *ABIL EM 90 (Evonik Industries AG) | |

### Beispiel 24 Kationische O/W Formulierung gemäß Tabelle 22:

**Tabelle 22: Formulierung gemäß Beispiel 24**

| | |
|---|---|
| Distearyldimonium Chlorid* | 1,00% |
| Polyglyceryl-3 Methylglucose Distearate** | 2,00% |
| Glyceryl Stearate*** | 2,00% |
| Stearyl Alcohol**** | 1,00% |
| Cetyl Ethylhexanoate***** | 9,00% |
| Caprylic/Capric Triglyceride****** | 10,00% |
| Siliconmethacrylat-Partikel aus Bsp. 1 | 1,50% |
| Glycerin | 3,00% |
| Wasser | ad 100,00% |
| Methylparaben, Ethylparaben, Methylisothiazolinone | 0,80% |
| Parfum | q.s. |

| | |
|---|---|
| *VARISOFT® TA 100 (Evonik Industries AG) **TEGO® Care 450 (Evonik Industries AG) ***TEGIN® M Pellets (Evonik Industries AG) ****TEGO® Alkanol 18 (Evonik Industries AG) *****TEGOSOFT® CO (Evonik Industries AG) ******TEGOSOFT® CT (Evonik Industries AG) | |

## Patentansprüche

1. Verfahren umfassend die Schritte:
a) Erzeugen einer Emulsion aus einer wässrigen Phase und einer organischen Phase, wobei die organische Phase mindestens einen Radikalstarter und mindestens ein Siloxan aufweist, das mindestens eine (Meth-)Acrylat-Gruppe der Formel (I)
-O-C(O)-CR=CH₂ (I)
mit R = -H oder -CH₃, aufweist,
unter Zugabe zumindest eines Festkörperemulgators und Mischen der beiden Phasen, wobei die organische Phase die innere Phase der Emulsion bildet, und
b) Auspolymerisieren der inneren Phase in Gegenwart eines Radikalstarters,
der in der organischen Phase besser löslich ist als in der wässrigen Phase, **dadurch gekennzeichnet, dass** das Siloxan ein mittleres molares Verhältnis von Gruppen der Formel (I) zu Si Atomen von kleiner 0,1 aufweist.

2. Verfahren nach Anspruch 1, **dadurch gekennzeichnet, dass** das Siloxan der Formel (II) genügt 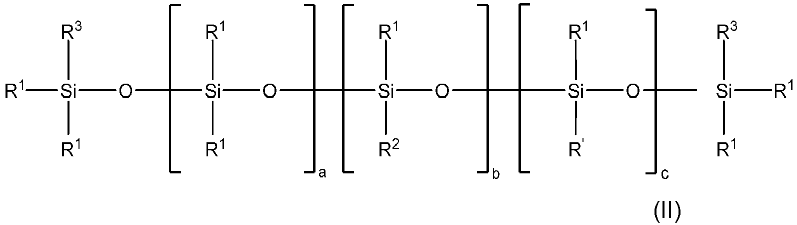 mit
R¹ = gleiche oder verschiedene Alkylreste, vorzugsweise Methylreste,
R² = ein Rest ungleich R¹ und R³, der Kohlenstoffatome aufweist,
R³ = gleiche oder verschiedene Reste R¹ oder Reste, die eine Gruppe gemäß Formel (I) aufweisen,
R'= gleiche oder verschiedene Reste, die eine Gruppe gemäß Formel (I) aufweisen,
a = 9 bis 250,
b = 0 bis 20,
c = 0 bis 20,
und der Maßgabe, dass pro Siloxan der Formel (II) wenn c = 0 mindestens ein Rest R³ vorhanden ist, der eine Gruppe gemäß Formel (I) aufweist.

3. Verfahren nach Anspruch 1 oder 2, **dadurch gekennzeichnet, dass** der Radikalstarter in einer Konzentration von 0,05 bis 2 Gew.-% bezogen auf die innere Phase zugefügt wird.

4. Verfahren nach mindestens einem der Ansprüche 1 bis 3, **dadurch gekennzeichnet, dass** der Festkörperemulgator eine mittlere Partikelgröße d₅₀ von > 100 nm bis 200 nm aufweist.

5. Verfahren nach mindestens einem der Ansprüche 1 bis 3, **dadurch gekennzeichnet, dass** als Festkörperemulgatoren solche eingesetzt werden, die ausgewählt sind aus der Gruppe der Metalloxide, Mischoxide, Nitride, Hydroxide, Carbonate und Silikate, die zumindest (teil-)hydrophobiert sind mit mindestens einer Verbindung aus der Gruppe der Silane, Siloxane, quaternären Ammoniumverbindungen, kationischen Polymere und Fettsäuren oder deren Anionen.

6. Verfahren gemäß zumindest einem der Ansprüche 1 bis 5, **dadurch gekennzeichnet, dass** die Siloxane, die mindestens eine (Meth-)Acrylat-Gruppe der Formel (I) aufweisen, diese in Form eines Restes aus der Gruppe der Reste 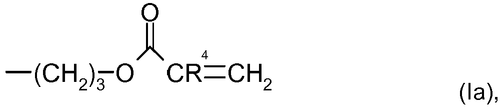 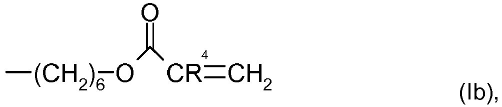 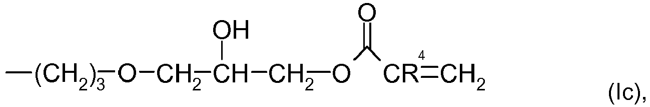 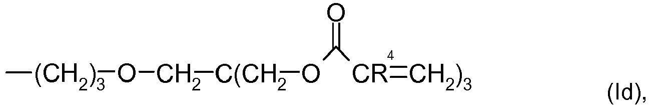 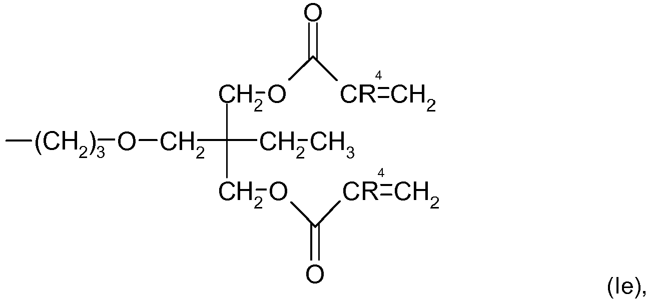 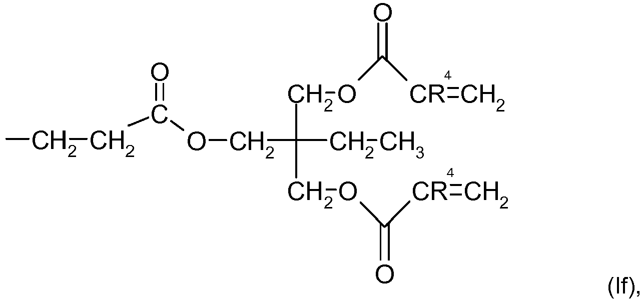 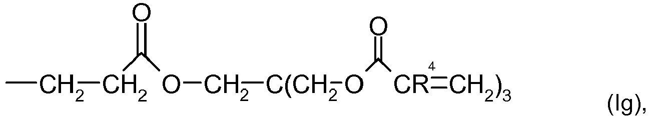 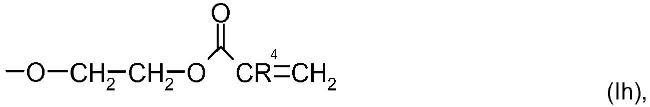 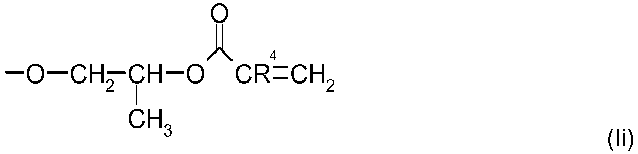 und/oder 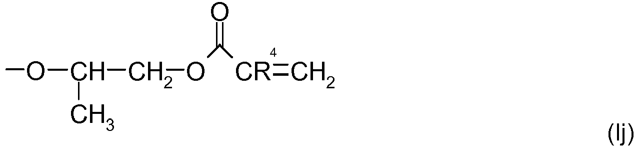 aufweisen, wobei R⁴ Wasserstoff oder eine Methylgruppe ist, vorzugsweise Wasserstoff ist.

7. Verfahren gemäß Anspruch 6, **dadurch gekennzeichnet, dass** die Siloxane, die mindestens eine (Meth-)Acrylat-Gruppe der Formel (I) aufweisen, diese in Form eines Restes ausgewählt aus der Gruppe umfassend die Reste mit den Formeln (Ib) und (Ie) aufweisen.

8. Verfahren gemäß zumindest einem der Ansprüche 2 bis 7, **dadurch gekennzeichnet, dass** mindestens ein Siloxan der Formel (II) eingesetzt wird, bei dem a einen Wert von 50 bis 220 einnimmt.

9. Verfahren gemäß zumindest einem der Ansprüche 2 bis 8, **dadurch gekennzeichnet, dass** mindestens ein Siloxan der Formel (II) eingesetzt wird, bei dem c einen Wert von 4 bis 12 einnimmt oder c = 0 ist und alle Reste R³ solche Reste sind, die eine Gruppe gemäß Formel (I) aufweisen.

10. Verfahren gemäß zumindest einem der Ansprüche 1 bis 9, **dadurch gekennzeichnet, dass** die organische Phase Substanzen beinhaltet, welche aus den Partikeln freigesetzt werden können.

11. Partikel, erhältlich durch Polymerisation eines Siloxans, das mindestens eine (Meth-)Acrylat-Gruppe der Formel (I)
-O-C(O)-CR=CH₂ (I)
mit R = -H oder -CH₃, aufweist, gemäß einem Verfahren gemäß einem der Ansprüche 1 bis 10, **dadurch gekennzeichnet, dass** das Siloxan ein mittleres molares Verhältnis von Gruppen der Formel (I) zu Si Atomen von kleiner 0,1 aufweist.

12. Partikel gemäß Anspruch 11, **dadurch gekennzeichnet, dass** die Partikel eine mittlere Partikelgröße d₅₀ von 1 bis 40 µm, bevorzugt von 3 bis 20 µm, besonders bevorzugt 5 bis 15 aufweisen.

13. Partikel gemäß einem der Ansprüche 11 oder 12, **dadurch gekennzeichnet, dass** die Partikel eine Partikelgröße d₉₀ von 10 bis 100 µm, bevorzugt von 15 bis 80 µm, besonders bevorzugt 40 bis 70 µm aufweisen.

14. Partikel nach mindestens einem der Ansprüche 11 bis 13, **dadurch gekennzeichnet, dass** die Partikel eine Partikelgröße d₁₀ von 0,5 bis 15 µm, bevorzugt von 1 bis 10 µm, besonders bevorzugt 2 bis 7,5 µm aufweisen.

15. Partikel nach mindestens einem der Ansprüche 11 bis 14, **dadurch gekennzeichnet, dass** die Partikel eine Kern-Schale-Struktur aufweisen, wobei die Schale durch partikuläre Emulgatoren gebildet wird, die einen inneren Kern aus Silicon(meth-)acrylat umschließt.

16. Partikel gemäß zumindest einem der Ansprüche 11 bis 15, **dadurch gekennzeichnet, dass** die Partikel monomere (Meth)acrylatverbindungen (Verbindungen die eine freie Gruppe der Formel (I) aufweisen) in einer Konzentration von weniger als 1500 Massen-ppm aufweisen.

17. Verwendung der Partikel gemäß zumindest einem der Ansprüche 11 bis 16 oder hergestellt gemäß zumindest einem der Ansprüche 1 bis 10 allein oder in Mischung mit weiteren Partikeln, Pigmenten und/oder weiteren üblichen Zusatzstoffen in Form von Pulvern oder Dispersionen in kosmetischen oder pharmazeutischen Zubereitungen oder in Pflegemitteln.

18. Zusammensetzungen enthaltend Partikel gemäß den Ansprüchen 11 bis 16 oder hergestellt gemäß einem der Ansprüche 1 bis 10.

19. Zusammensetzung gemäß Anspruch 18, **dadurch gekennzeichnet, dass** die Zusammensetzung eine Dispersion von Partikeln in wässrigen oder organischen Medien ist, wobei der Dispersion ggf. ein Dispergierhilfsmittel, ein Tensid und/oder ein Verdicker zugesetzt sein kann.

20. Zusammensetzung nach Anspruch 18 oder 19, **dadurch gekennzeichnet, dass** die Zusammensetzung eine kosmetische oder pharmazeutische Zubereitung oder ein Pflegemittel ist.

## Claims

1. Process comprising the steps:
a) producing an emulsion of an aqueous phase and an organic phase, where the organic phase has at least one radical initiator and at least one siloxane which has at least one (meth)acrylate group of the formula (I)
-O-C(O)-CR=CH₂ (I)
where R = -H or -CH₃,
with the addition of at least one solid-body emulsifier and mixing of the two phases, where the organic phase forms the inner phase of the emulsion, and
b) fully polymerizing the inner phase in the presence of a radical initiator which is more soluble in the organic phase than in the aqueous phase,
**characterized in that** the siloxane has an average molar ratio of groups of the formula (I) to Si atoms of less than 0.1.

2. Process according to Claim 1, **characterized in that** the siloxane satisfies the formula (II): 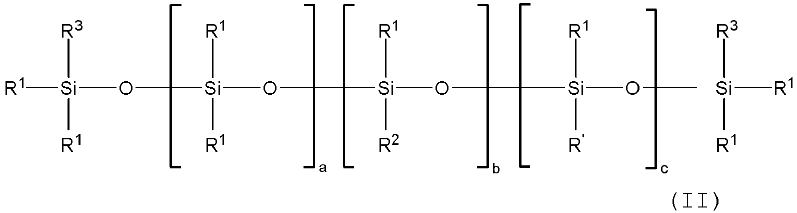 where
R¹ = identical or different alkyl radicals, preferably methyl radicals,
R² = a radical which is not the same as R¹ and R³ and has carbon atoms,
R³ = identical or different radicals R¹ or radicals which have a group according to formula (I),
R'= identical or different radicals which have a group according to formula (I),
a = 9 to 250,
b = 0 to 20,
c = 0 to 20,
and the proviso that, per siloxane of the formula (II), if c = 0, at least one radical R³ is present which has a group according to formula (I).

3. Process according to Claim 1 or 2, **characterized in that** the radical initiator is added in a concentration of 0.05 to 2% by weight, based on the inner phase.

4. Process according to at least one of Claims 1 to 3, **characterized in that** the solid-body emulsifier has an average particle size d₅₀ of > 100 nm to 200 nm.

5. Process according to at least one of Claims 1 to 3, **characterized in that** the solid-body emulsifiers used are those which are selected from the group of metal oxides, mixed oxides, nitrides, hydroxides, carbonates and silicates which are at least (partially)hydrophobicized with at least one compound from the group of silanes, siloxanes, quaternary ammonium compounds, cationic polymers and fatty acids or anions thereof.

6. Process according to at least one of Claims 1 to 5, **characterized in that** the siloxanes which have at least one (meth)acrylate group of the formula (I) have this group in the form of a radical from the group of the radicals 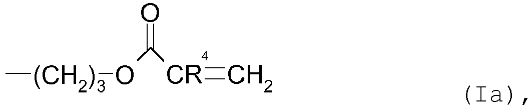 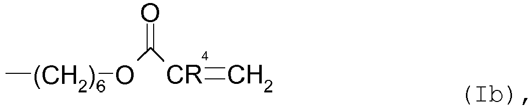 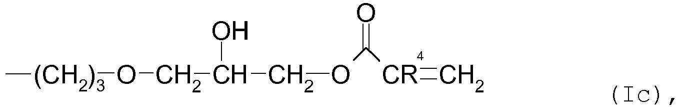 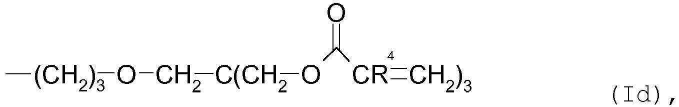 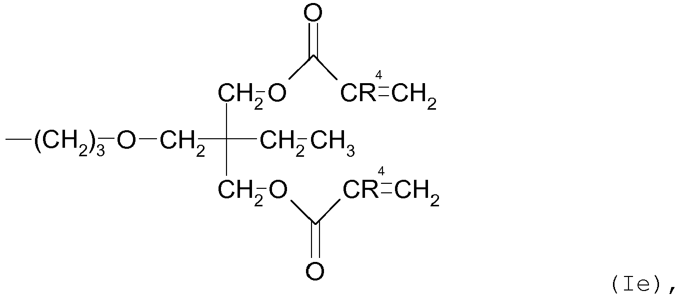 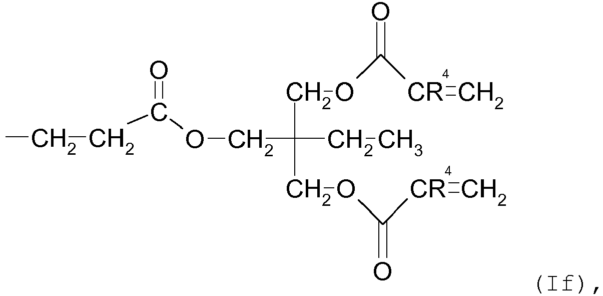 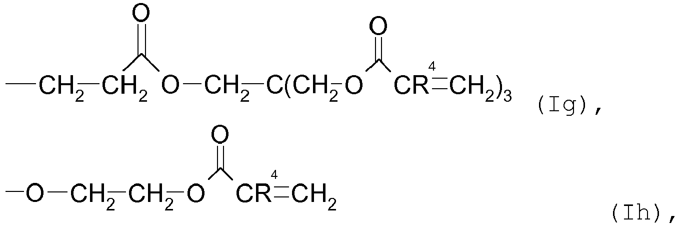 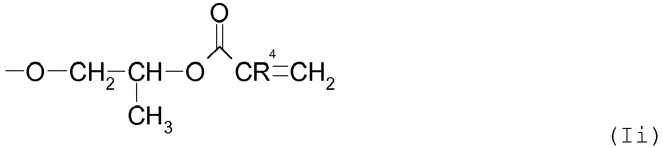 and/or 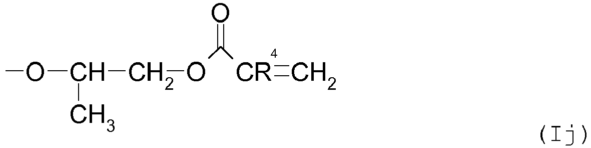 where R⁴ is hydrogen or a methyl group, preferably hydrogen.

7. Process according to Claim 6, **characterized in that** the siloxanes which have at least one (meth)acrylate group of the formula (I) have this in the form of a radical selected from the group comprising the radicals with the formulae (Ib) and (Ie) .

8. Process according to at least one of Claims 2 to 7, **characterized in that** at least one siloxane of the formula (II) is used in which a assumes a value from 50 to 220.

9. Process according to at least one of Claims 2 to 8, **characterized in that** at least one siloxane of the formula (II) is used in which c assumes a value from 4 to 12 or c = 0 and all of the radicals R³ are those radicals which have a group according to formula (I).

10. Process according to at least one of Claims 1 to 9, **characterized in that** the organic phase contains substances which can be released from the particles.

11. Particles obtainable by polymerization of a siloxane which has at least one (meth)acrylate group of the formula (I)
-O-C(O)-CR=CH₂ (I)
where R = -H or -CH₃, according to a process according to one of Claims 1 to 10,
**characterized in that** the siloxane has an average molar ratio of groups of the formula (I) to Si atoms of less than 0.1.

12. Particles according to Claim 11, **characterized in that**
the particles have an average particle size d₅₀ of 1 to 40 µm, preferably of 3 to 20 µm, particularly preferably 5 to 15.

13. Particles according to one of Claims 11 and 12, **characterized in that** the particles have a particle size d₉₀ of 10 to 100 µm, preferably from 15 to 80 µm, particularly preferably 40 to 70 µm.

14. Particles according to at least one of Claims 11 to 13, **characterized in that** the particles have a particle size d₁₀ of 0.5 to 15 µm, preferably from 1 to 10 µm, particularly preferably 2 to 7.5 µm.

15. Particles according to at least one of Claims 11 to 14, **characterized in that** the particles have a core/shell structure, where the shell is formed by particulate emulsifiers and surrounds an inner core of silicone (meth)acrylate.

16. Particles according to at least one of Claims 11 to 15, **characterized in that** the particles have monomeric (meth)acrylate compounds (compounds which have a free group of the formula (I)) in a concentration of less than 1500 ppm by mass.

17. The use of the particles according to at least one of Claims 11 to 16 or produced according to at least one of Claims 1 to 10 alone or in a mixture with further particles, pigments and/or further customary additives in the form of powders or dispersions in cosmetic or pharmaceutical preparations or in care compositions.

18. Compositions comprising particles according to Claims 11 to 16 or produced according to one of Claims 1 to 10.

19. Composition according to Claim 18, **characterized in that** the composition is a dispersion of particles in aqueous or organic media, where optionally a dispersion auxiliary, a surfactant and/or a thickener can be added to the dispersion.

20. Composition according to Claim 18 or 19, **characterized in that** the composition is a cosmetic or pharmaceutical preparation or a care composition.

## Revendications

1. Procédé comprenant les étapes suivantes :
a) la formation d'une émulsion à partir d'une phase aqueuse et d'une phase organique, la phase organique comprenant au moins un démarreur radicalaire et au moins un siloxane, qui comprend au moins un groupe (méth)acrylate de formule (I)
-O-C(O)-CR=CH₂ (I)
avec R = -H ou -CH₃,
avec ajout d'au moins un émulsifiant solide et le mélange des deux phases, la phase organique formant la phase intérieure de l'émulsion, et
b) la polymérisation de la phase intérieure en présence d'un démarreur radicalaire, qui est mieux soluble dans la phase organique que dans la phase aqueuse,
**caractérisé en ce que** le siloxane présente un rapport molaire moyen entre les groupes de formule (I) et les atomes Si de moins de 0,1.

2. Procédé selon la revendication 1, **caractérisé en ce que** le siloxane satisfait la formule (II) 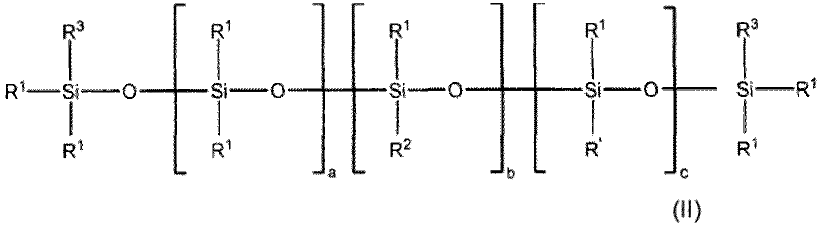 avec
R¹ = des radicaux alkyle identiques ou différents, de préférence des radicaux méthyle,
R² = un radical différent de R¹ et R³, qui comprend des atomes de carbone,
R³ = des radicaux R¹ identiques ou différents, ou des radicaux qui comprennent un groupe selon la formule (I),
R' = des radicaux identiques ou différents, qui comprennent un groupe selon la formule (I),
a = 9 à 250,
b = 0 à 20,
c = 0 à 20,
et à condition qu'au moins un radical R³ qui comprend un groupe selon la formule (I) soit présent par siloxane de formule (II) lorsque c = 0.

3. Procédé selon la revendication 1 ou 2, **caractérisé en ce que** le démarreur radicalaire est ajouté en une concentration de 0,05 à 2 % en poids, par rapport à la phase intérieure.

4. Procédé selon au moins l'une quelconque des revendications 1 à 3, **caractérisé en ce que** l'émulsifiant solide présente une taille de particule moyenne d₅₀ de > 100 nm à 200 nm.

5. Procédé selon au moins l'une quelconque des revendications 1 à 3, **caractérisé en ce que** des émulsifiants solides qui sont choisis dans le groupe constitué par les oxydes métalliques, les oxydes mixtes, les nitrures, les hydroxydes, les carbonates et les silicates, qui sont au moins (partiellement) hydrophobés avec au moins un composé du groupe constitué par les silanes, les siloxanes, les composés d'ammonium quaternaires, les polymères cationiques et les acides gras ou leurs anions, sont utilisés.

6. Procédé selon au moins l'une quelconque des revendications 1 à 5, **caractérisé en ce que** les siloxanes qui comprennent au moins un groupe (méth)acrylate de formule (I) comprennent celui-ci sous la forme d'un radical du groupe constitué par les radicaux suivants : 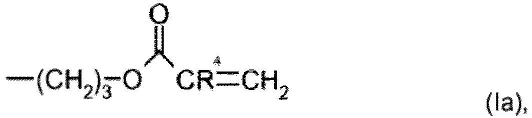 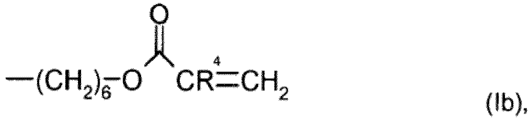 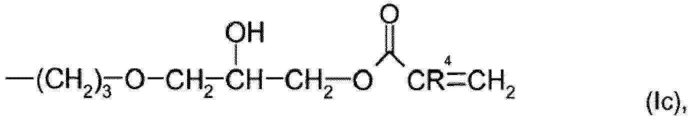 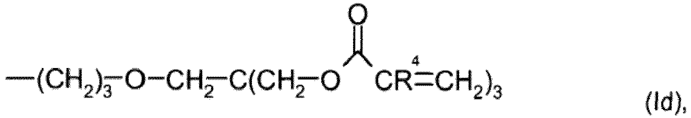 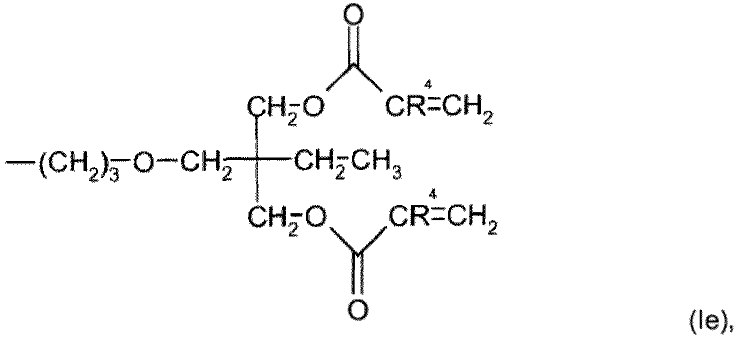 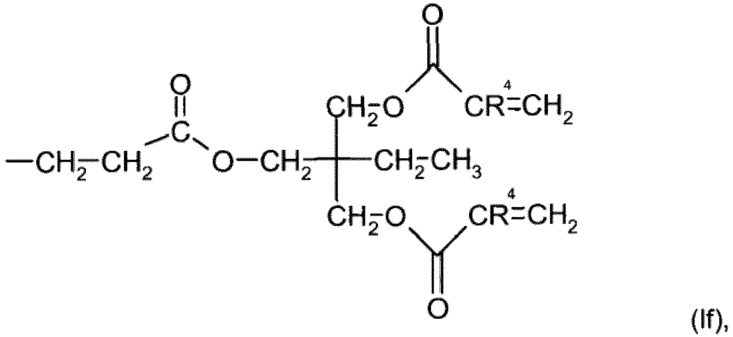 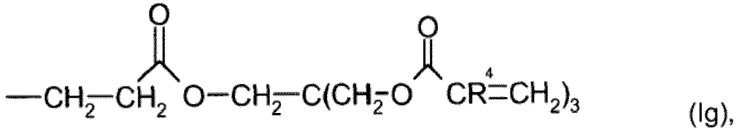 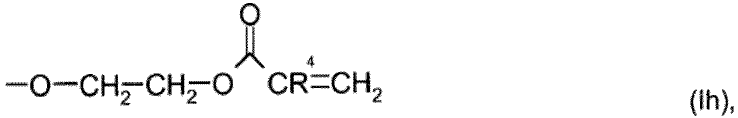 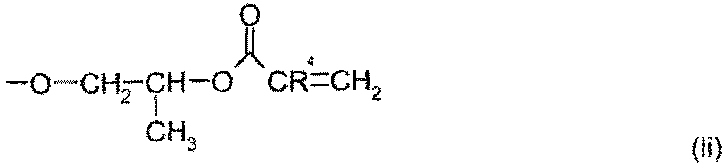 et/ou 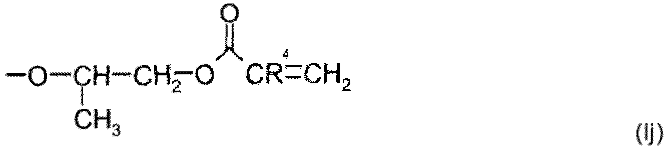 R⁴ représentant l'hydrogène ou un groupe méthyle, de préférence l'hydrogène.

7. Procédé selon la revendication 6, **caractérisé en ce que** les siloxanes qui comprennent au moins un groupe (méth)acrylate de formule (I) comprennent celui-ci sous la forme d'un radical choisi dans le groupe comprenant les radicaux des formules (Ib) et (Ie).

8. Procédé selon au moins l'une quelconque des revendications 2 à 7, **caractérisé en ce qu'**au moins un siloxane de formule (II) est utilisé, dans lequel a prend une valeur de 50 à 220.

9. Procédé selon au moins l'une quelconque des revendications 2 à 8, **caractérisé en ce qu'**au moins un siloxane de formule (II) est utilisé, dans lequel c prend une valeur de 4 à 12 ou c = 0, et tous les radicaux R³ sont des radicaux qui comprennent un groupe selon la formule (I).

10. Procédé selon au moins l'une quelconque des revendications 1 à 9, **caractérisé en ce que** la phase organique contient des substances qui peuvent être libérées par les particules.

11. Particules, pouvant être obtenues par polymérisation d'un siloxane, qui comprend au moins un groupe (méth)acrylate de formule (I)
-O-C(O)-CR=CH₂ (I)
avec R = -H ou -CH₃, par un procédé selon l'une quelconque des revendications 1 à 10, **caractérisées en ce que** le siloxane présente un rapport molaire moyen entre les groupes de formule (I) et les atomes Si de moins de 0,1.

12. Particules selon la revendication 11, **caractérisées en ce que** les particules présentent une taille de particule moyenne d₅₀ de 1 à 40 µm, de préférence de 3 à 20 µm, de manière particulièrement préférée de 5 à 15 µm.

13. Particules selon l'une quelconque des revendications 11 ou 12, **caractérisées en ce que** les particules présentent une taille de particule d₉₀ de 10 à 100 µm, de préférence de 15 à 80 µm, de manière particulièrement préférée de 40 à 70 µm.

14. Particules selon au moins l'une quelconque des revendications 11 à 13, **caractérisées en ce que** les particules présentent une taille de particule d₁₀ de 0,5 à 15 µm, de préférence de 1 à 10 µm, de manière particulièrement préférée de 2 à 7,5 µm.

15. Particules selon au moins l'une quelconque des revendications 11 à 14, **caractérisées en ce que** les particules présentent une structure noyau-enveloppe, l'enveloppe étant formée par des émulsifiants particulaires, qui entourent un noyau intérieur en (méth)acrylate de silicone.

16. Particules selon au moins l'une quelconque des revendications 11 à 15, **caractérisées en ce que** les particules comprennent des composés de (méth)acrylate monomères (composés qui comprennent un groupe libre de formule (I)) en une concentration de moins de 1 500 ppm en masse.

17. Utilisation des particules selon au moins l'une quelconque des revendications 11 à 16 ou fabriquées selon au moins l'une quelconque des revendications 1 à 10 seules ou en mélange avec d'autres particules, des pigments et/ou d'autres additifs usuels sous la forme de poudres ou de dispersions dans des préparations cosmétiques ou pharmaceutiques ou dans des agents de soin.

18. Compositions contenant des particules selon les revendications 11 à 16 ou fabriquées selon l'une quelconque des revendications 1 à 10.

19. Composition selon la revendication 18, **caractérisée en ce que** la composition est une dispersion de particules dans des milieux aqueux ou organiques, un adjuvant de dispersion, un tensioactif et/ou un épaississant pouvant éventuellement être ajoutés à la dispersion.

20. Composition selon la revendication 18 ou 19, **caractérisée en ce que** la composition est une préparation cosmétique ou pharmaceutique ou un agent de soin.
